# FASCICULE DE BREVET EUROPEEN

(11) **EP 1 045 856 B1**
(45) Date de publication et mention de la délivrance du brevet: **09.04.2003**
(21) Numéro de dépôt: 99900500.2
(22) Date de dépôt: 07.01.1999
(51) Int. Cl.: C07H 17/075, A61K 31/70

(54) **NOUVEAUX AMIDES AROMATIQUES, LEUR PROCEDE DE PREPARATION ET LEUR APPLICATION COMME MEDICAMENTS**
AROMATISCHE AMIDE, IHRER VERFAHREN ZUR HERSTELLUNG UNG VERWENDUNG ALS ARZNEIMITTELN
NOVEL AROMATIC AMIDES, PREPARATION METHOD AND APPLICATION AS MEDICINES

(30) Priorité: 08.01.1998 FR 9800116; 15.10.1998 FR 9812936
(43) Date de publication de la demande: 25.10.2000
(73) Titulaire: Aventis Pharma S.A., 92160 Antony (FR)
(72) Inventeur: HAESSLEIN, Jean-Luc, F-77181 Courtry (FR); KLICH, Michel, F-93250 Villemomble (FR); LAURIN, Patrick, F-93100 Montreuil (FR); MUSICKI, Branislav, F-75013 Paris (FR); PERIERS, Anne-Marie, F-77230 Moussy-le-Neuf (FR)
(86) Numéro de dépôt international: FR9900014
(87) Numéro de publication internationale: WO99035155

(56) Documents cités:
- WO-A-97/47634
- US-A- 4 226 978

## Description

La présente invention concerne de nouveaux amides aromatiques, leur procédé de préparation et leur application comme médicaments.

L'invention a pour objet les composés de formule (I) : 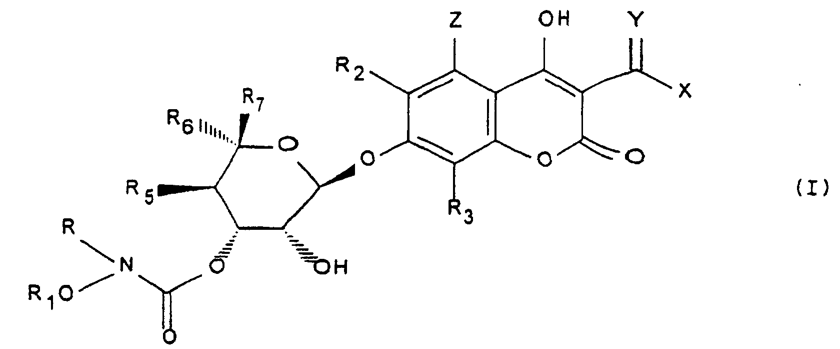 dans laquelle
- Y représente un atome d'oxygène, ou un radical N-Nalc₁ ou NOalc₂ dans lequel alc₁ et alc₂ représentent un radical alkyle, renfermant jusqu'à 12 atomes de carbone éventuellement interrompu par un ou plusieurs atomes d'oxygène, de soufre ou d'azote éventuellement substitué par un ou plusieurs atomes d'halogène, par un radical aryle éventuellement substitué par un ou plusieurs atomes d'halogène, par un radical hétérocyclique, par un ou plusieurs radicaux 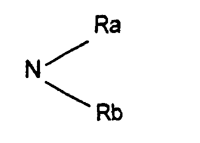
dans lequel Ra et Rb identiques ou différents l'un de l'autre représentent un atome d'hydrogène, un radical alkyle renfermant jusqu'à 8 atomes de carbone éventuellement substitué, ou Ra et Rb forment ensemble avec l'atome d'azote auxquels ils sont joints un hétérocycle pouvant renfermer en outre un autre hétéroatome choisi parmi l'oxygène, le soufre ou l'azote,

X représente un atome d'hydrogène, un radical hydroxyle, un radical alkyle, alkényle ou alkynyle éventuellement interrompu par un ou plusieurs atomes d'oxygène, de soufre et ou d'azote, linéaire, ramifié ou cyclique renfermant jusqu'à 12 atomes de carbone, éventuellement substitué par un ou plusieurs atomes d'halogène, par un radical hétérocyclique, un ou plusieurs radicaux OH libres ou estérifiés, C≡N, NO₂, 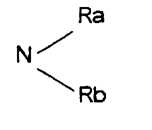 dans lequel Ra et Rb, identiques
ou différents, représentent un atome d'hydrogène, un radical alkyle renfermant jusqu'à 8 atomes de carbone, ou Ra et Rb forment avec l'atome d'azote auquel ils sont liés un hétérocycle renfermant éventuellement un autre hétéroatome choisi parmi l'azote, le soufre ou l'oxygène, ou X représente un radical alkoxy ou un radical 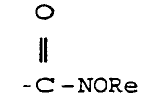 dans lequel Re représente un radical alkyle renfermant jusqu'à 8 atomes de carbone, éventuellement substitué par un ou plusieurs des substituants indiqués ci-dessus,
ou X représente un radical NRcRd dans lequel Rc et Rd identiques ou différents, représentent un atome d'hydrogène ou un radical alkyle renfermant jusqu'à 12 atomes de carbone, éventuellement substitué par un ou plusieurs des substituants indiqués ci-dessus, ou Rc et Rd forment ensemble avec l'atome d'azote auquel ils sont liés un hétérocycle renfermant éventuellement un autre hétéroatome choisi parmi l'azote, le soufre ou l'oxygène,
- Z représente un atome d'hydrogène ou d'halogène ou un radical OH libre, éthérifié ou estérifié,
- R₂ représente un atome d'hydrogène ou d'halogène,
- R₃ représente un atome d'hydrogène, un radical alkyle renfermant jusqu'à 8 atomes de carbone ou un atome d'halogène,
- R représente un atome d'hydrogène ou un radical alkyle renfermant jusqu'à 4 atomes de carbone,
- R₁ représente un atome d'hydrogène, un radical alkyle, alkényle ou alkynyle linéaire, ramifié ou cyclique renfermant jusqu'à 8 atomes de carbone, éventuellement substitué par un
   ou plusieurs atomes d'halogène, un radical C≡N, un radical aryle renfermant jusqu'à 14 atomes de carbone,
- R₅ représente un atome d'hydrogène, un radical O-alkyle renfermant jusqu'à 4 atomes de carbone,
- ou bien R₆ représente un radical alkyle ou CH₂-O-alkyle,
   dans lequel alkyle représente un radical alkyle renfermant jusqu'à 8 atomes de carbone, éthényle ou 2-propényle,
- R₇ représente un atome d'hydrogène ou un radical alkyle renfermant jusqu'à 8 atomes de carbone,
- ou bien R₆ et R₇ forment avec l'atome de carbone qui les portent, un cycle renfermant jusqu'à 8 atomes de carbone, ainsi que les sels du composé de formule (I), lorsque les composés de formule (I) ont une fonction basique.

Des dérivés apparentés à ceux répondant à la formule (I) ci-dessus ont été décrits dans la demande WO 97/47634. Ces dérivés se distinguent essentiellement de ceux de la présente demande au niveau du substituant en position 3' du cycle ribose, lequel est un groupement O-CO-R₄, dans lequel R₄ peut notamment être un radical NRgRf, Rg et Rf étant Hydrogène, alkyle, aryle ou hétéroaryle ou formant un hétérocycle avec l'atome d'azote auquel ils sont liés. R₄ ne peut représenter un reste formant avec le carbonyle un reste de type hydroxamique substitué ou non.

Comme exemples de sels on peut également citer les sels formés avec les acides acétique, propionique, trifluo-roacétique, maléique, tartrique, méthanesulfonique, benzènesulfonique, paratoluènesulfonique, chlorhydrique, bromhydrique, iodhydrique, sulfurique, phosphorique et spécialement les acides stéariques, éthylsuccinique ou laurylsulfonique.

Dans la définition des substituants :
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclo-hexyle,
- l'halogène est de préférence le fluor ou le chlore, ou le brome,
- le radical aryle est de préférence le radical phényle,
- le radical hétérocyclique est de préférence le radical pyrrolyle, pyrrolidinyle, pyridyle, pyrazinyle, pyrimidyle, pipéridinyle, pipérazinyle, quinuclidinyle, oxazolyle, isoxa-zolyle, morpholinyle, indolyle, imidazolyle, benzimidazolyle, triazolyle, thiazolyle, azétidinyle, aziridinyle.

L'invention a plus particulièrement pour objet les composés de formule (I) dans lesquels Y représente un atome d'oxygène, ceux dans lesquels Y représente un radical NO-alkyle dans lequel le radical alkyle renferme jusqu'à 4 atomes de carbone, par exemple ceux dans lesquels Y représente le radical NOC₂H₅.

Parmi les composés préférés de l'invention on peut citer les composés de formule (I) dans lesquels X représente un radical alkyle renfermant jusqu'à 4 atomes de carbone et notamment le radical CH₃, ou encore ceux dans lesquels X représente un radical NH₂, ou encore ceux dans lesquels X représente le radical :

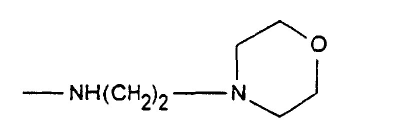

Parmi les composés préférés de l'invention, on peut citer les composés de formule (I) dans lesquels R₁ représente un radical :

HC≡C-CH₂-

ceux dans lesquels R représente un atome d'hydrogène, ou encore ceux dans lesquels R₃ représente un radical méthyle, ou encore ceux dans lesquels Z représente un atome d' hydrogène, ou encore ceux dans lesquels R₂ représente un atome d'hydrogène, ou encore ceux dans lesquels R₅ représente un radical OCH₃, ou encore ceux dans lesquels R₆ représente un radical méthyle, ou encore ceux dans lesquels R₇ représente un radical méthyle, ceux dans lesquels R₇ représente un radical éthyle, ceux dans lesquels R₆ et R₇ forment avec le carbone qui les porte un radical cyclopentyle.

Parmi les composés préférés de l'invention, on peut citer les composés dont la préparation est donnée ci-après dans la partie expérimentale et tout particulièrement les composés des 1, 2, 3, 4, 5 et 9.

Les produits de formule générale (I) possèdent une très bonne activité antibiotique sur les bactéries gram telles que les staphylocoques, les streptocoques, les pneumocoques, les entérocoques, listeria, anaérobies.

Les composés de l'invention peuvent donc être utilisés comme médicaments dans le traitement des infections à germes sensibles et, notamment, dans celui des staphylococcies, telles que les septicémies à staphylocoques, staphylococcies malignes de 1 a face ou cutanées, pyodermites, plaies septi - ques ou suppurantes, furoncles, anthrax, phlegmons, érysipèles et acné, staphylococcies telles que les angines aigües primitives ou post-grippales, broncho-pneumonie, suppuration pulmonaires, les streptococcies telles que les angines aigües, les otites, les sinusites, la scarlatine, les pneumococcies telles que les pneumonies, les bronchites et la diphtérie. Les produits de la présente invention sont également actifs contre les infections dues à des germes comme Haemophilus influenzae.

L'invention a donc pour objet les composés de formule (I) à titre de médicaments.

L'invention a plus spécialement pour objet à titre de médicaments les composés indiqués ci-dessus comme composés préférés.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale ou injectable.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 3000 mg par jour par voie orale ou injectable, chez l'adulte pour les produits préférés .

L'invention a également pour objet un procédé de préparation des composés de formule (I), caractérisé en ce que l'on soumet un composé de formule (II) : 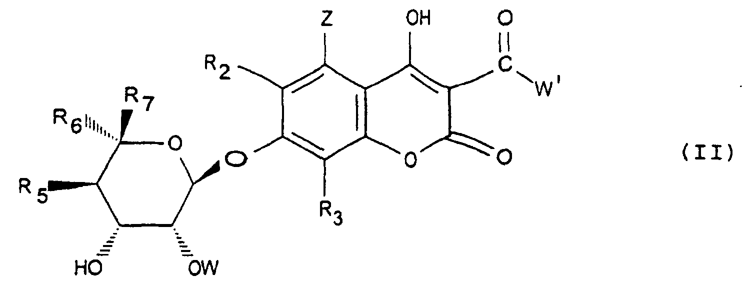 dans laquelle les radicaux R₂, R₃, Z, R₅, R₆ et R₇ conservent leur signification précédente, OW représente un groupement hydroxyle bloqué et W' représente un radical alkyle ou Oalkyle renfermant jusqu'à 4 atomes de carbone,
- à l'action un agent susceptible d'introduire le radical 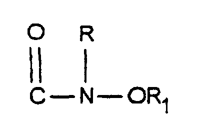
ou d'une suite d'opérations susceptibles d'introduire le radical 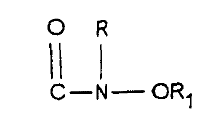 R et R₁ conservant leur signification précédente,
- à l'action d'un agent susceptible de libérer le radical hydroxyle du radical OW,
- à l'action éventuelle d'un agent susceptible de remplacer W' par le radical X différent de alkyle ou Oalkyle,
- à l'action éventuelle d'un agent susceptible d'introduire le radical Y différent de l'oxygène,
- à l'action éventuelle d'un agent de salification.

Les produits de formule (II) utilisés au départ du procédé de l'invention sont des produits nouveaux, la préparation de certains produits de formule (II) est donnée ci-après dans la partie expérimentale.

Les autres produits de formule (II) peuvent être synthétisés par analogie avec les procédés décrits dans la partie expérimentale.

L ' invention a plus spécialement pour objet les composés de formule (II) dont la préparation est donnée dans la partie expérimentale.

Dans un mode de réalisation préféré :

L ' introduction du radical 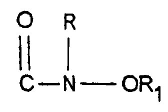 se fait en plusieurs étapes, d'abord action d'un phénylchloroformiate substitué ou non, puis action d'un composé de formule R₁ONHR
dans laquelle R₁ et R conservent leur signification précédente,
le groupe OH est bloqué sous la forme d'un tétra-hydropyrane,
la libération de l'hydrolyse se fait par hydrolyse acide, par exemple par action de l'acide paratoluène sulfonique,
la transformation éventuelle du radical W' en radical X et la transformation du radical Y se fait selon les procédés classiques. Pour le radical Y, il s'agit notamment de l'action d'une amine.

L'invention a également pour objet un procédé caractérisé en ce que le produit de formule (II) est préparé par action d'un composé de formule (III) 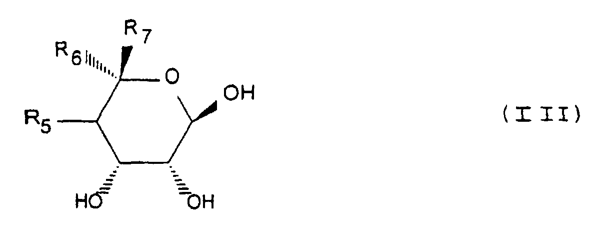 dans lequel R₅, R₆ et R₇ conservent leur signification précédente sur un composé de formule (IV) 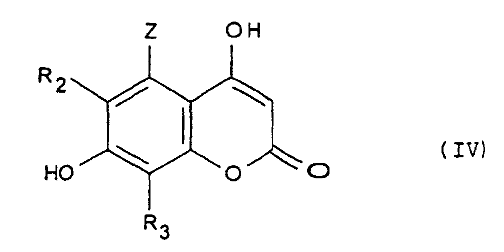 dans lequel R₂, R₃ et Z conservent leur signification précédente, puis d'un agent de blocage du radical hydroxyle libre.

Les composés de formule (III) suivants sont nouveaux et sont en eux-mêmes un objet de la présente invention, à savoir :

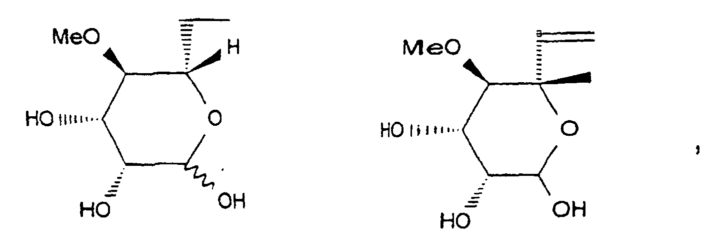

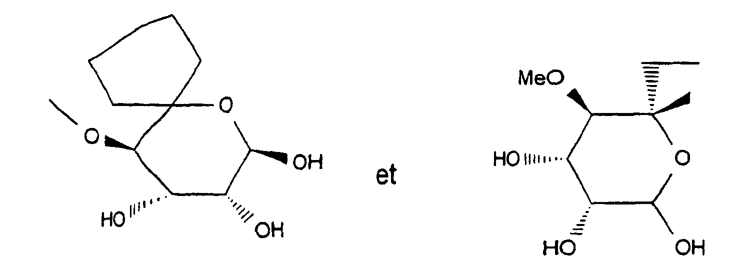

Les exemples suivants illustrent l'invention sans toutefois la limiter.

### Préparation 1 : 7-[[6-déoxy-5-C-méthyl-4-O-méthyl-2-O-(tétrahydro-2H-pyran-2-yl)-alpha-L-lyxo-hexopyranosyl]oxy]-4-hydroxy-8-méthyl-2-oxo-2H-1-benzopyran-3-carboxylate d'éthyle

### STADE A : 7-[(6-déoxy-5-C-méthyl-4-O-méthyl-alpha-L-lyxo-hexopyranosyl)oxy]-8-méthyl-2-oxo-4-(phénylméthoxy)-2H-1-benzopyran-3-carboxylate d'éthyle

On agite sous atmosphère d'argon, une solution renfermant 80 g de 7-hydroxy-8-méthyl-2-oxo-4-(phénylméthoxy)-2H-1-benzopyran-3-carboxylate d'éthyle dans 1200 ml de chlorure de méthylène. On ajoute à 0°C 52,07 g de 6-déoxy-5-C-méthyl-4-0-méthyl-L-lyxo-hexopyranose et 71,22 g de triphényl -phosphine.

On introduit à 0°C, 54,78 ml d' azocarboxylate de diisopropyle. Au bout d'une heure de réaction à température ambiante, on ajoute à nouveau 34 g de triphénylphosphine et 25,6 ml d'azocarboxylate de diisopropyle. On agite 16 heures à la température ambiante. On évapore à demi, filtre la suspension en éluant avec le mélange toluène/alcool isopropylique (95-5). Lorsque le produit commence à passer, on poursuit avec un mélange à 6% d'alcool isopropylique. On obtient après empâtage dans 700 ml de mélange hexane/acétate d'éthyle (4-1), 64, 4 g de produit recherché que l'on utilise tel quel dans le stade suivant.

### STADE B : 7-[(6-déoxy-5-C-méthyl-4-O-méthyl-3-O-(triéthyl-silyl)-alpha-L-lyxo-hexopyranosyl)oxy]-8-méthyl-2-oxo-4-(phenylméthoxy)-2H-1-benzopyran-3-carboxylate d' éthyle

On agite sous argon à la température ambiante 50 g d'une solution du stade A dans 500 ml de chlorure de méthylène. On ajoute 42 ml de diisopropyléthylamine et 9,66 g d'imidazole. La solution est agitée 15 minutes ou refroidie à 0°C, ajoute en 30 minutes, goutte à goutte, 20, 64 ml de triéthylchlorosilane et agite pendant 2 heures à 0°C. On verse le milieu réactionnel dans une solution molaire de dihydrogénophosphate de sodium. On extrait au chlorure de méthylène. On sèche et évapore à sec. On recueille 66,27 g de produit que l'on purifie sur silice en éluant avec un mélange de chlorure de méthylène à 0,75% d'acétone. Lorsque le produit est pratiquement isolé, on élue avec une solution de chlorure de méthylène à 1 % d'acétone. On obtient après empâtage dans un mélange hexane/acétate d'éthyle (9-1) 41,04 g de produit recherché.
RMN 1H (300 MHz, CDCl3, ppm)
0,73 (q, 6H), 1,04 (t, 9H), 1,04 (s, 3H), 1,30 (s, 3H), 1,40 (t, 3H) , 2,24 (s, 3H) , 2,74 (d, J=1 Hz, 1H mobile), 3,28 (d, 1H, J=9), 3,53 (s, 3H), 4,05 (m, 1H) , 4,27 (dd, 1H, J=3, 5 et 9 Hz) , 4,43 (q, 2H) , 5,31 (s, 2H), 5,62 (d , 1H, J=2 Hz) , 7,12 (d, 1H, J=9 Hz) , 7,43 (m, 5H) , 7,63 (d, 1H, J=9 Hz).

### STADE C : 7-[[6-déoxy-5-C-méthyl-4-O-méthyl-2-O-(tétrahydro-2H-pyran-2-yl)-3-O-(triéthylsilyl)-alpha-L-lyxo-hexopyranosyl]oxy]-8-méthyl-2-oxo-4-(phénylméthoxy)-2H-1-benzopyran-3-carboxylate d'éthyle

On agite sous argon une solution renfermant 40,9 g de produit du stade précédent dans 400 ml de chlorure de méthylène à la température ambiante. On ajoute quelques gouttes d'acide paratoluène sulfonique, puis 11,54 ml de dihydropyrane.
On agite 2 heures à la température ambiante. On ajoute 6 g de bicarbonate de sodium. On agite la suspension pendant 15 minutes . On dilue avec 1000 ml d'un mélange hexane/acétate d'éthyle (2-1) et verse sur de l'eau. On décante, sèche la phase organique sur sulfate de sodium et évapore à sec. On obtient 54,67 g de produit que l'on purifie en éluant avec un mélange hexane/acétate d'échyle (4-1). On obtient ainsi 36, 83 g de produit.

### STADE D : 7-[[6-déoxy-5-C-méthyl-4-O-méthyl-2-O-(tétrahydro-2H-pyran-2-yl)-3-O-(triéthylsilyl)-alpha-L-lyxo-hexopyra-nosyl]oxy]-4-hydroxy-8-méthyl-2-oxo-2H-1-benzopyran-3 - carboxylate d'éthyle

On hydrogène 18 g de produit préparé au stade précédent, en solution dans 360 ml de tétrahydrofuranne en présence de 0 , 240 g de palladium sur charbon. On filtre. On lave le catalyseur avec un peu de tétrahydrofuranne. On évapore 100 ml de solvant et obtient une solution que l'on utilise telle quelle dans le stade suivant.

### STADE E : 7-[[6-déoxy-5-C-méthyl-4-O-méthyl-2-O-(tétrahydro-2H-pyran-2-yl)-alpha-L-lyxo-hexopyranosyl]oxy]-4-hydroxy-8-méthyl-2-oxo-2H-1-benzopyran-3-carboxylate d'éthyle

On refroidit sous argon à 0°C une solution renfermant 15,31 g de produit du stade précédent dans 250 ml de tétrahydrofuranne. On ajoute goutte à goutte 31 ml de tétrabutylammonium fluorure (1M dans THF). On dilue le milieu réactionnel avec 400 ml d'un mélange hexane/acétate d'éthyle (1-2). On ajoute 300 ml d'une solution à 10% d'hydrogénosulfate de sodium et décante On sèche et évapore à sec. On solubilise le produit brut obtenu dans 20 ml d'éther éthylique. On refroidit à -10°C
et ajoute sous agitation 80 ml de pentane. On agite la suspension obtenue à -20°C, filtre à -16°C. On lave le produit obtenu avec du pentane et sèche. On obtient 9,4 g de produit recherché.

### Préparation 2 : 3-acétyl-7-[[6-déoxy-5-C-méthyl-4-O-méthyl-2-O-(tétrahydro-2H-pyran-2-yl)alpha-L-lyxo-hexopyranosyl]oxy]-4 -hydroxy-8-méthyl-2H-1-benzopyran-2-one

### STADE A : 4-(diphénylméthoxy)-8-méthyl-7-(tétrahydro-2H-pyran-2-yl)-2H-1-benzopyran-2-one

On introduit 55 g de 4-hydroxy-8-méthyl-7- (tétrahydro-2H-pyran-2-yl)-2H-1-benzopyran-2-one dans 250 ml de diméthylformamide anhydre chauffé à 40°C, et ajoute goutte à goutte, une solution de 58,3 g de diphényldiazométhane dans 250 ml de diméthyl formamide. L'addition est faite en 3 heures en maintenant la température à 40°C.

On ajoute à nouveau plusieurs portions de 3 g de diphényldiazométhane et agite une heure a 40°C.

On verse le milieu réactionnel sur 2 1 d'éther sulfurique. On lave la solution organique avec une solution aqueuse de bicarbonate de sodium, avec une solution de soude (0,1 M), à l'eau et à la saumure. On évapore à sec. Le résidu est agité dans un mélange éther isopropyl ique-hexane (1-2). On essore et sèche l'insoluble. On obtient 20,5 g de produit recherché
CCM CH₂Cl₂-AcOEt (95-5) . Rf = 0,44.

### STADE B : 4-(diphénylméthoxy)-7-hydroxy-8-méthyl-2H-1-benzopyran-2-one

On ajoute 35 ml d'une solution d'acide chlorhydrique 0,9 M dans le méthanol, dans une solution renfermant un mélange de 20 g de produit du stade A, 100 ml de dichloromethane et 100 ml de méthanol. On agite 2 heures à la température ambiante et évapore les solvants. On reprend le résidu dans de l'éthanol absolu refroidi à 0°C. On essore l'insoluble et rince à l'alcool glacé puis à l'éther sulfurique. On sèche et recueille 15,53 g de produit que l'on reprend dans l'éther, essore et sèche. On obtient 14, 54 g de produit recherché.
RMN 1H (300 MHz, CDCl₃, ppm)
2,31 (s, 3H) , 5,62 (s, 1H) , 6,35 (s, 1H) , 6,78 (d, 1H, J= Hz), 7,75 (d, 1H, J= _Hz), 6,99 à 7,10 (m, _H) , 7,30 à 7,42 (m, _H).

### STADE C : 7-[(6-déoxy-5-C-méthyl-4-O-méthyl-alpha-L-lyxo-hexopyranosyl)oxy]-4-(diphénylméthoxy)-8-méthyl-2H-1-benzopyran-2-one

On refroidit à 0°C un mélange de 91,13 g de produit du stade B, 58,6 g de 6-déoxy-5-C-méthyl-4-méthyl-O-méthyl-L-lyxo-hexopyranose et 80 g de triphénylphosphine dans 900 ml de dichlorométhane. On ajoute goutte à goutte 60 ml de diisopropylazodicarboxylate. On agite pendant 1 heure à la température ambiante.

On ajoute 34 g de triphénylphosphine et 25 ml de diisopropylazodicarboxylate. On agite 1 heure à la tempéra ture ambiante. On ajoute 34 g de triphénylphosphine et 25 ml de diisopropylazodicarboxylate et agite 12 heures à la température ambiante. On concentre sous pression réduite. On. chromatographie en éluant avec un mélange toluène/alcool isopropylique (95-5). Après réunion des fractions et évaporation des solvants, on recueille après recristallisation dans l'éther isopropylique, 86, 83 g de produit recherché.
RMN 1H (300 MHz, CDCl₃, ppm)
1,13 (s, 3H), 1,37 (s, 3H), 2,24 (s, 3H), 2,69 (s, 1H) , 2,79 (s, 1H) , 3,38 (d, 1H, J= 10 Hz) , 3, 60 (s, 3H) , 4,24 (m, 1H), 4,28 (m, 1H) , 5,56 (s, 1H) , 5,64 (d, 1H, J=1,5 Hz) , 6,35 (s, 1H), 7,18 (d, 1H), 7,81 (d, 1H) , 7, 39 (m, 10 H).

### STADE D : 7- [ [6-déoxy-5-C-méthyl-4-O-méthyl-3-O-(triéthyl-silyl)-alpha-L-lyxo-hexopyranosyl]oxy]-4-(diphénylméthoxy)-8-méthyl-2H-1-benzopyran-2 - one

On ajoute 26,6 g d'imidazole et 70,15 ml de diisopropyléthylamine dans une solution refroidie à 0°C, renfermant 80 g de produit du stade précédent et 60 0 ml de dichlorométhane. On ajoute goutte à goutte 33,5 ml de chlorure de triéthyl-silyle. On agite 1 heure à la tempéra-ture ambiante. On lave avec une solution aqueuse de dihydr-o-génophosphate de sodium (1M), à l'eau et à la saumure. On sèche sur du sulfate de magnésium, filtre et concentre. On recueille 97,58 g de produit que l'on purifie par chromato-graphie sur silice en éluant avec le mélange dichlorométhane acétone (de 0,8 à 1%). On obtient 46,5 g de produit.
RMN 1H (300 MHz, CDCl₃-d6, ppm)
0,60 (q, _H, J=_Hz), 0,74 (q, _H, J=_Hz), 0, 97 (t, _H, J=_Hz), 1,00 (t, _H, J=_Hz), 1,10 (s, 3H), 1,32 (s, 3H), 2, 24 (s, 2H), 2,74 (s, 1H), 3,31 (d, 1H, J=_Hz), 3,54 (s, 3H), 4,07 (m, 1H), 4,29 (dd, 1H, J=_Hz), 5,50 (s, 1H), 5,64 (d, 1H, J=_Hz, 6,35 (s, 1H), 7,28 (d, 1H, J=_Hz), 7,81 (d, 1H, J=_Hz), 7,40 (m).

### STADE E : 7-[[6-déoxy-5-C-méthyl-4-O-méthyl-2-O-(tétrahydro-2H-pyran-2-yl)-3-O-(triéthylsilyl)-alpha-L-lyxo-hexopyra nosyl]oxy]-4-(diphénylméthoxy)-8-méthyl-2H-1-benzopyran-2-one

On ajoute 19 ml de dihydropyrane et 400 mg d'acide paratoluène sulfonique (APTS) dans une solution renfermant 67 g de produit du stade précédent et 1 l de dichloro-méthane. On agite pendant 40 minutes à la température ambiante, on ajoute 300 mg d'APTS. Après 30 minutes, on ajoute 100 mg d'APTS, puis encore 100 mg d'APTS. On agite pendant 20 minutes supplémentaires, puis introduit de l'hydrogénocarbonate de sodium finement broyé. On agite pendant 10 minutes, dilue le milieu réactionnel avec un mélange hexane/acétate d'éthyle (1-2), lave à l'eau et à la saumure. On sèche. On filtre et évapore les solvants. On chromatographie le produit obtenu en éluant avec le mélange heptane/acétate d'éthyle (4-1). On recueille 77,9 g de produit recherché.
RMN 1H (300 MHz, DMSO-d6, ppm)
0,64 (q, _H, J=_Hz), 0,73 (q, _H, J=_Hz), 0,95 à 1,32 (_H), 2, 25 (s, _H) , 2,27 (s, _H), 3,30 (d, _H, J=_Hz), 3,4 (d, _H, J=_ Hz), 3,50 (m, 2H), 3,93 (m, 2H), 3,53 (s, _H), 3,54 (s, _H), 4,04 à 4,15, 4,36 (dd, _H, J=_Hz), 4,94 (1), 4,96 (1), 5, 50 (sl, _H), 5,65 (sl), 6,37 (s, 1H), 7,15 (d, _H, J= _Hz) , 7, 19 (d, _H, J=_Hz), 7,81 (m, 1H), 7,30 à 7,44, 1,47 à 2,0O.

### STADE F : 7-[[6-déoxy-5-C-méthyl-4-O-méthyl-2-O-(tétrahydro-2H-pyran-2-yl)-3 -O-triméthylsilyl)-alpha-L-lyxo-hexopyranosyl]oxy]-4-hydroxy-8-méthyl-2H-1-benzopyran-2-one

On hydrogène en présence de palladium sur charbon (2 g, 10 %), 15 g du produit du stade précédent dans 150 ml d' éthanol absolu. On élimine le catalyseur par filtration et évapore les solvants jusqu'à siccité.

On obtient 14,4 g de produit.

### STADE G : 3-acétyl-7-[[6-déoxy-5-C-méthyl-4-O-méthyl-2-0-(tétrahydro-2H-pyran-2-yl) -3-O-(triéthylsilyl)-alpha-L-lyxo-hexopyranosyl]oxy]-4-hydroxy-8-méthyl-2H-1-benzopyran-2-one

On ajoute dans une solution renfermant 14,37 g de produit du stade précédent et 150 ml de dichlorométhane, 6,52 g de diméthylaminopyridine. On introduit goutte à goutte 2,72 ml d'anhydride acétique. On agite à la tempéra-ture ambiante sous argon pendant 1 heure. On dilue avec 200 ml de dichlorométhane, lave avec une solution aqueuse de dihydro-génénophosphate de sodium. On sèche sur sulfate de magnésium, filtre et concentre. On obtient 14,9 g de produit recherché.

### STADE H : 3-acétyl-7-[[6-déoxy-5-C-méthyl-4-O-méthyl-2-O-(tétrahydro-2H-pyran-2-yl)-alpha-L-lyxo-hexopyranosyl]oxy]-4-hydroxy-8-méthyl-2H-1-benzopyran-2-one

On introduit goutte à goutte à 0°C, 27 ml d'une solution 1M de fluorure de tétrabutylammonium dans le tétrahydrofuranne, dans une solution renfermant 15,2 ml de produit du stade précédent dans 250 ml de THF. On agite sous argon pendant 48 heures à température ambiante. On dilue le milieu avec le mélange acétate d' éthyle/hexane, lave à l'eau et à la saumure. On sèche, filtre et concentre à sec. On obtient 13 g d'un produit que l'on triture dans le pentane, on élimine le surnageant et répète l'opération plusieurs fois. On maintient le produit à + 4°C, le broie en présence de pentane, filtre l'insoluble, rince et sèche . On obtient 6, 99 g de produit recherché.
RMN 1H (300 MHz, CDCl₃-d6, ppm)
1,09 (s, 3H) , 1,11 (s, 3H) , 1,35 (s, 3H) , 1,36 (s, 1H) , 1,50 à 1,90 (m, 8), 2,23 (s, 3H) , 2,24 (s, 3H) , 2,76 (s, 3H) , 3,28 (d, 1H, J=_Hz), 3,33 (d, 1H, J=_Hz) , 3,63 (s, 3H) , 3,64 (s, 3H) , 3,54 (m), 3,97 (m) , 4,07 (m), 4,20 à 4,30 (_, 2H), 4,59 (m, _H) , 4, 82 (m, _H), 5,63 (sl, _H) , 5,85 (sl, _H) , 7,20 (d, 1H, J=_Hz) , 7,88 (m, _H) .

### EXEMPLE 1 : Acide (2-propynyloxy)-carbamique 3'ester de 7-[[6-deoxy-5-C-méthyl-4-O-méthyl-alpha-L-lyxo-hexopyranosyl] oxy]-4-hydroxy-8-méthyl-2-oxo-2H-1-benzopyran-3 -carboxamide

### STADE A : 3- (4-nitrophénylcarbonate) de 7-[[6-deoxy-5-C-méthyl-4-0-méthyl-2-O-(tétrahydro-2H-pyran-2-yl)-alpha-L-lyxo-hexopyranosyl]oxy]-4-hydroxy-8-méthyl-2-oxo-2H-1-benzopyran-3-carboxylate d'éthyle

On met en solution sous argon dans 80 ml de chlorure de méthylène, 4 g du produit de la préparation 1. On ajoute 2,15 g de diméthylaminopyridine et à 0°C, 2 g de 4-nitro phénylchloroformate. On agite pendant 1 heure à 0°C. On évapore le chlorure de méthylène et obtient le produit recherché.

### STADE B : 7- [[6-déoxy-5-C-méthyl-4-O-méthyl-3-O-[[(2-propynyloxy)amino]carbonyl]-2-O-(tétrahydro-2H-pyran-2-yl) alpha-L-lyxo-hexopyranosyl]oxy]-4-hydroxy-8-méthyl-2-oxo-2H-1-benzopyran-3-carboxylate du éthyle

On met en solution 1,215 g de chlorohydrate O-propargylhydroxylamine, dans 40 ml de diméthylformamide. A 0°C, on ajoute 0,392 g d'hydrure de sodium (à 50% d'huile) et agite pendant une heure à cette température. On introduit à 0°C dans cette suspension 4 ml de solution du produit préparé au stade précédent dans le diméthylformamide et 940 mg de diméthylaminopyridine. On agite pendant 1 heure à 0°C. On dilue avec un mélange hexane/ acétate d'éthyle (1-2). On lave la solution organique avec 400 ml de solution d' hydrogénosulfate de sodium à 10%, sèche sur sulfate de sodium et évapore à sec. On obtient 7,87 g de produit recherché brut.

### STADE C : Acide (2-propynyloxy)-carbamique 3'-ester de 7-[[6-déoxy-5-C-méthyl-4-O-méthyl-2-O-(tétrahydro-2H-pyran-2-yl) alpha-L-lyxo-hexopyranosyl]oxy]-4-hydroxy-8-méthyl-2-oxo-2H-1-benzopyran-3-carboxamide

On met en solution dans 50 ml de tétrahydrofuranne 2 g de produit du stade précédent. On sature la solution obtenue avec de l' ammoniaque à 0°C pendant 10 minutes et agite 48 heures à la température ambiante. On dilue avec 100 ml d'un mélange hexane/acétate d'éthyle (1-1). La solution organique est lavée avec 100 ml d'une solution de dihydrogénophosphate de sodium à 1M, puis elle est séchée sur sulfate de magnésium et évaporée à sec. On obtient 2 g du produit recherché.

### STADE D :Acide (2-propynyloxy)-carbamique 3'ester de 7-[[6-deoxy-5-C-méthyl-4-O-méthyl-alpha-L-lyxo-hexopyranosyl] oxy]-4-hydroxy-8-méthyl-2-oxo-2H-1-benzopyran-3-carboxamide

On met en solution dans 20 ml de méthanol 2 g du produit du stade précédent et 200 mg d'acide p-toluène-sulfonique. On agite pendant 1 heure . On dilue avec 100 ml de mélange hexane/acétate d'éthyle (1-1). On lave avec une solution saturée de dihyrogénophosphate de sodium, sèche et amène à sec. On obtient 1,6 g de produit que l'on purifie en éluant avec un mélange chlorure de méthylène/méthanol 8%. On empâte avec un mélange éther éthylique/pentane. On obtient 0,574 g de produit recherché .
RMN 1H (300 MHz, DMSO-d6, ppm)
1,04 (s, 3H) , 1,26 (s, 3H) , 2,20 (s, 3H) , 3,45, (s, 3H), 3,52 (d, 1H) , 3,56 (m, 1H) , 4,14 (m, 1H) , 4,46 (m, 2H) , 5,20 (m, 1H) , 5,59 (sl, 1H) , 5,77 (d, 1H mobile), 7,22 (d, 1Hz) , 7,83 (d, 1H) , 8,71 (m) et 8,96 (m) (2H mobiles).

### EXEMPLE 2 : Acide (2-propynyloxy)-carbamique 3' -ester de 7-[(6-déoxy- 5-C-méthyl-4-O-méthyl-alpha-L-lyxo-hexopyranosyl) oxy]-4-hydroxy-8-méthyl-N-[2-(4-morpholinyl)éthyl]-2-oxo-2H-1-benzopyran-3 - carboxamide

### STADE A : Acide (2-propynyloxy)carbamique 3'-ester de 7-[[6-déoxy-5-C-méthyl-4-O-méthyl-2-O-(tértrahydro-2H-pyran-2-yl)-alpha-L-lyxo-hexopyranosyl]oxy]-4-hydroxy-8-méthyl-N-[2-(4-morpholinyl)éthyl]-2-oxo-2H-1-benzopyran-3-carboxamide

On introduit 7,5 ml de 2-(4-morpholino)éthylamine dans une solution renfermant 1 g de produit du stade B de l'exemple 1 dans 4 ml de tétrahydrofuranne. On agite 24 heures à la température ambiante. On dilue avec 100 ml d'hexane/acétate d'éthyle/tétrahydrofuranne (1-4-1). On lave avec une solution saturée de dihydrogénophosphate de sodium, sèche sur sulfate de magnésium et évapore à sec . On obtient 1 g de produit recherché.

### STADE B : Acide (2-propynyloxy)-carbamique 3'-ester de 7-[(6-déoxy-5-C-méthyl-4-O-méthyl-alpha-L-lyxo-hexopyranosyl) oxy]-4-hydroxy-8-méthyl-N-[2-(4-morpholinyl)éthyl]-2-oxo-2H-1-benzopyran- 3 - carboxamide

On met en solution 0,97 mmoles du produit du stade précédent dans 10 ml de méthanol. On ajoute 100 mg d'acide p-toluènesulfonique. On agite pendant 1 heure à la température ambiante. On ajoute à nouveau 80 mg d'acide p-toluènesulfonique. On agite pendant 3 heures. On dilue avec 50 ml de mélange hexane/acétate d'éthyle (1-3 ) . On lave avec 75 ml d'une solution 1M de dihydrogénophosphate de sodium, sèche sur sulfate de magnésium et évapore à sec. On purifie le produit en le chromatographiant sur silice en éluant avec le mélange chlorure de méthylène/méthanol (91 - 9). On empâte le produit obtenu dans un mélange éther éthylique/pentane. On obtient 0,150 g de produit recherché.
RMN 1H (300 MHz, DMSO-d6, ppm)
1,03 (s, 3H), 1,27 (s, 3H), 2,21 (s, 3H) , 2,50 (masqué, 4H) , 2,57 (t, 2H) , 3,45 (s, 3H) , de 3,40 à 3, 69 (m, 4H) , 4,13 (m, 1H), 4,47 (d, 2H, J=2,5 Hz), 5,20 (dd, 1H, J=3 et 10 Hz), 5,60 (d, 1H, J=2 Hz) , 5,77 (d, 1H, J=5 Hz), 7,21 (d, 1H, J=9 Hz), 7,84 (d, 1H, J=9 Hz) , 9,45 (t, 1H mobile) , 10,72 (m, 1H mobile).

### EXEMPLE 3 : acide (2-propynyloxy)-carbamique-3'-ester de 7-[[6-déoxy-5-C-méthyl-4-0-méthyl-alpha-L-lyxo-hexopyranosyl] oxy]-4-hydroxy-3-[1-(méthoxyimino)éthyl]-8-méthyl-2H-1-benzopyran-2-one

### STADE A : 7-[6-déoxy-5-C-méthyl-4-O-méthyl-2-O-(tétrahydro-2H-pyran-2-yl)-alpha-L-lyxo-hexopyranosyl]oxy]-4-hydroxy-3-[1-(méthoxyimino)éthyl]-8-méthyl-2H-1-benzopyran-2-one

On chauffe à 40°C en présence de 0,597 g d'acétate de potassium et de 0,407 mg de chlorhydrate de O-méthylhydroxylamine, une solution renfermant 1,2 g du produit de la préparation 2. On agite pendant une heure et demie à 40°C le milieu réactionnel, le dilue avec un mélange acétate d'éthyle/hexane (4-1), lave avec 150 ml d'une solution d' hydrogénophosphate de sodium. On rince à l'eau, sèche, filtre et évapore à sec.

### STADE B : 3-(4-nitrophénylcarbonate) de 7-[[6-déoxy-5-C-méthyl- 4 -O-méthyl-2-O-(tétrahydro-2H-pyran-2-yl)-alpha-L-lyxo-hexopyranosyl]oxy] -4-hydroxy-3-[1-(méthoxyimino)éthyl]-8 -méthyl-2H-benzopyran-2-one

On ajoute 0,390 g de diméthylaminopyridine dans une solution renfermant 1,28 mmoles du produit du stade précédent et 12 ml de dichlorométhane. On ajoute 0,319 g de 4-nitrophényl chloroformate. On agite 30 minutes à 0°C. On évapore le chlorure de méthyle et sèche le produit obtenu. On obtient ainsi 1,218 mmoles de produit recherché.

### STADE C : acide (2-propynyloxy)-carbamique 3'-ester de 7- [[6-déoxy-5-C-méthyl-4-O-méthyl-2-O-(tétrahydro-2H-pyran-2-yl ) alpha-L-lyxo-hexopyranosyl]oxy]-4-hydroxy-3-[1-(méthoxy imino)éthyl]-8-méthyl-2H-1-benzopyran-2-one

A une solution refroidie à 0°C de 0,655 g de chlorhydrate de O-propargylhydroxylamine dans 6 ml de diméthylformamide, on ajoute 0,240 g d'hydrure de sodium (à 55% d'huile minérale) . On agite pendant 30 minutes à 0°C et verse dans une solution renfermant 1,218 mmoles du produit du stade précédent et 6 ml de DMF en présence de 0,150 g de diméthylaminopyridine. Au bout d'une heure à 0°C, on verse le mélange réactionnel dans un mélange acétate d'éthyle/hexane à 20%, lave avec une solution d'hydrogénosulfate de sodium à 10%, à l'eau et à la saumure. On sèche et évapore les solvants à sec. On obtient 0,865 g de produit recherché.

### STADE D : acide (2-propynyloxy)-carbamique-3'-ester de 7-[[6-déoxy-5-C-méthyl-4-0-méthyl-alpha-L-lyxo-hexopyranosyl]oxy]-4-hydroxy-3-[1- (méthoxyimino)éthyl]-8-méthyl-2H-1-benzopyran-2-one

A une solution renfermant 1,218 mmoles du produit du stade précédent et 12 ml de méthanol, on ajoute 150 mg d'APTS. On agite une heure à température ambiante. On dilue avec un mélange acétate d'éthyle/hexane (1-1) et lave avec une solution aqueuse de dihydrogénophosphate de sodium 1M, puis à la saumure. On sèche la phase organique sur sulfate de magnésium. On évapore les solvants jusqu'à siccité. On chromatographie le produit obtenu en éluant avec le mélange dichlorométhane/acétone (85-15), et recueille 0,394 g de produit que l'on redissout dans l'éther et précipite au pentane. On isole l'insoluble par filtration et sèche sous pression réduite. On obtient ainsi 0,380 g de produit recherché.

### EXEMPLE 4 : Acide (2-propynyloxy)-carbamique 3'-ester de 7 - [(6-déoxy-5-C-méthyl-4-O-méthyl-alpha-L-lyxo-hexopyranosyl) oxy]-3-[1-(éthoxyimino)éthyl]-4-hydroxy-8-méthyl-2H-1- benzopyran-2-one

En opérant comme précédemment, on a obtenu le produit recherché .
RMN CDCl₃ ppm
1,17 (s) - 1,38 (s) : 2 CH3 Gem ; 1,38 (t) : 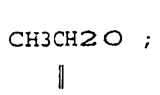 1,61 (s) : 4 mobile, 2,25 (s) - 2,53 (s) : 2CH3-C- ; 2,57 (t) : J=2,5 h-C C- ; 2,64 (sl) OH-CH ; 3,54 (s) : OCH3 ; 3,61 (d) : J=9, 5 H4 rex ; 4,23 (q) CH3-CH2-O en léger dé faut ; 4,43 (sl) : H2eq ; 4,57 (d) : 2H OCH2-C CH ; 5,46 (dd) : J=2,5 et 9,5 H3 ox ; 5,61 (d) : J=2,5 H1 eq ; 7,12 (d) H'6 ; 7,77 (d) : H'5 ; H mobiles 7,78 : 14,15 et 15,11 Absorptions le long du spectre
2,05 (acétone), 4,13, 4,75-4,60-15,67.

### EXEMPLE 5 : [7R-(7.alpha.,8.bêta.,9.bêta.,10.alpha.)]-(2-propynyloxy)-carbamate de 8-hydroxy-7-[4-hydroxy-3-[1-(méthoxyimino)éthyl]-8-méthyl-2-oxo-2H-1-benzopyran-7-yl]-10-méthoxy-6-oxaspiro[4.5]décan-9-yle

La préparation de ce produit et celle des produits de départ mis en oeuvre peut-être schématisée comme suit :

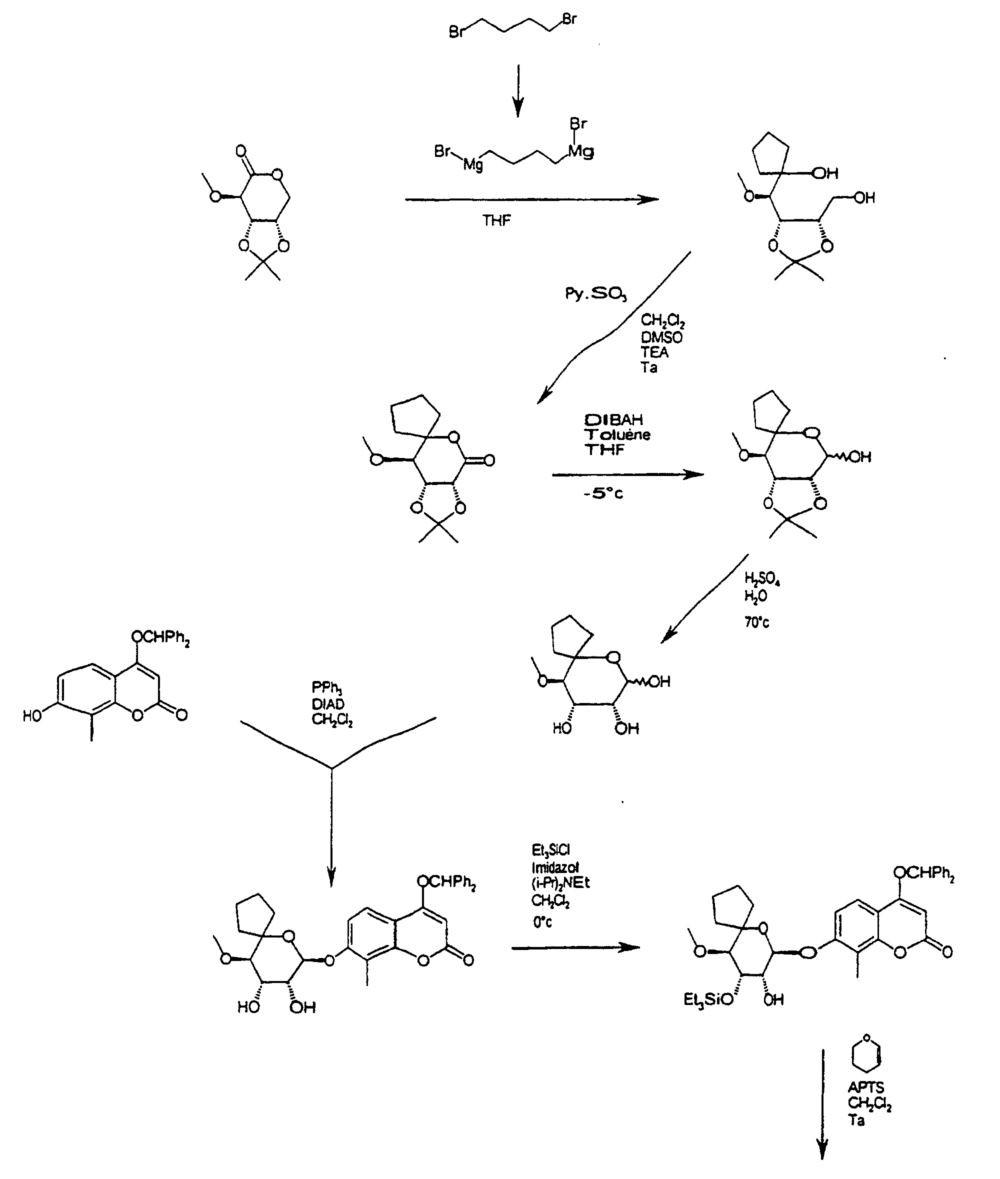

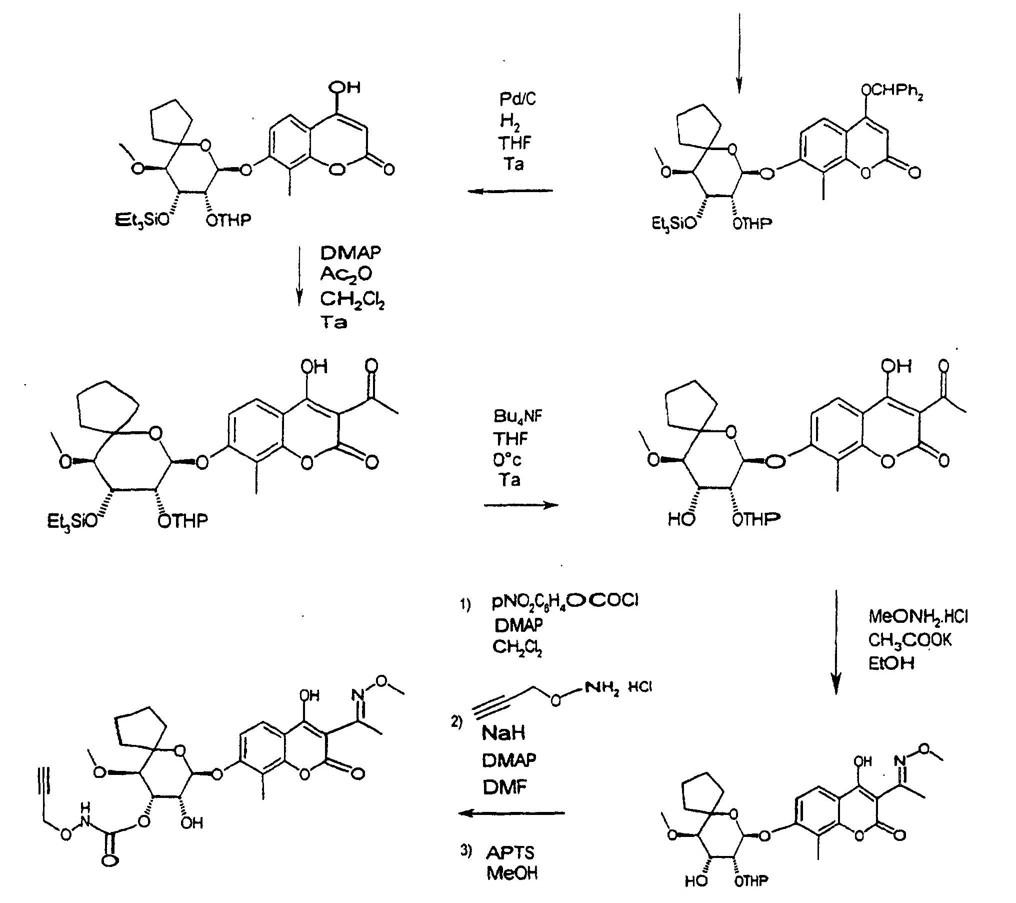

### Préparation 3 : [8R- (8.alpha.,9.alpha,10.bêta)]-10-méthoxy-6-oxaspiro[4.5]décane-7,8,9-triol

### STADE A : [4S-[4.alpha.,5.alpha.(S*)]]-2,2-diméthyl-5-[(1-hydroxycyclopentyl)méthoxyméthyl]-1,3-dioxolane-4-méthanol

On introduit 20 ml d'une solution de dibromobutane (106 ml de dibromobutane dans 200 ml de THF), dans un mélange renfermant 43 g de magnésium, 100 ml de THF et un cristal d'iode. On met sous ultrasons le mélange réactionnel. On ajoute 1,7 1 de THF. On ajoute le reste de la solution dibromée. On maintient l'agitation pendant 2 heures 30. On ajoute à 17°C une solution renfermant 80,37 g de delta -lactone de l'acide 2-0-méthyl-3,4-O-(1-méthyléthylidène) -L- arabinonique et 1 litre de THF . On agite environ 5 heures à température ambiante. On refroidit à 0°C, ajoute une solution saturée en chlorure d'ammonium. On décante, soutire la phase organique, extrait avec une solution d'acétate d'éthyle à 20% d'heptane . On lave, sèche et évapore à sec. On obtient 111,85 g de produit recherché.

### STADE B : [3'aS-(3'a.alpha.,7'.alpha,7'a.bêta.)]-7'-méthoxy-dihydro-spiro[cyclopentane-1,6'-[6H] -1, 3-dioxolo[4,5-c]pyran]-4'(3aH)-one

On ajoute 221 g de pyridine sulfurtrioxyde (PySO3) dans une solution renfermant 111 g du produit préparé au stade A et un mélange d'un litre de chlorure de méthylène, 1 litre de DMSO, 0,607 1 de triéthylamine. On agite 2 heures à la température ambiante. On verse sur une solution aqueuse de phosphate acide de sodium, extrait avec un mélange acétate d'éthyle, heptane (1-1). On sèche, filtre et évapore à sec. On obtient 57,7 g de produit recherché .

### STADE C : [8R-(8.alpha.,9.alpha,10.bêta)] -10-méthoxy-6-oxaspiro[4,5]decane-7,8,9-triol

On ajoute à -5°C, 157 ml d'une solution 1,5 M d'hydrure de dibutylaluminium dans le toluène dans une solution renfermant 56 g du produit du stade précédent et 300 ml de THF. On agite à -3°C pendant 1 heure. On ajoute 1 litre d'une solution 1 M de tartrate double de sodium et de potassium. On agite 15 minutes à la température ambiante. On extrait le milieu réactionnel avec un mélange acétate d'éthyle-heptane 1-1. On lave à l'eau, à la saumure, sèche et évapore à sec. On agite à 70°C le résidu obtenu en présence de 150 ml d'une solution d'acide sulfurique 0,1 N et 150 ml d'eau durant 2,5 heures. On refroidit à la température ambiante, ajoute du carbonate de baryum, et agite 1 heure à la température ambiante. On filtre, et évapore à sec. On obtient 49 g du produit recherché.

### EXEMPLE 5 : [7R-(7.alpha.,8.bêta.,9.beta.,10.alpha.)]-(2-propynyloxy)-carbamate de 8-hydroxy-7-[ 4 -hydroxy-3-[1-méthoxyimino)éthyl]-8-méthyl-2-oxo-2H-1-benzopyran-7-yl]-10-méthoxy-6-oxaspiro[4.5]décan-9-yle

### STADE A : [7R-(7.alpha.,8.bêta.,9.bêta.,10.alpha.)]-7-[(8,9-dihydroxy-10-méthoxy-6-oxaspiro[4.5]décan-7-yl)oxy]-4-(diphénylméthoxy)-8-méthyl-2H-1-benzopyran-2-one

On ajoute goutte à goutte à 0°C 45,30 g de diisopropylazodicarboxylate (DIAD) dans un mélange de 49 g du produit de la préparation 3, 73 g du produit du stade B de la préparation 2 à savoir le 4-(diphénylméthoxy)-7-hydroxy-8-méthyl-2H-1-benzopyran-2-one et 59 g de triphényl-phosphine. On agite 1,5 heure à la température ambiante. On ajoute à 0°C 1 équivalent de triphénylphosphine et de DIAD. On évapore les solvants, reprend à l'éther et obtient le produit recherché .

### STADE B : [7R-(7.alpha.,8.bêta.,9.bêta.,10.alpha.)]-4-(diphénylméthoxy)-7- [[8-hydroxy-10-méthoxy-9-[(triéthylsilyl)oxy]-6-oxaspiro[4.5]décan-7-yl)oxy]-8-méthyl-2H-1-benzopyran-2 -one

On ajoute à 0°C 15,21 g d'imidazole, 40,1 ml de diisopro-pylamine et 18,75 g de chlorure de trié thylsilane dans une solution renfermant 48 g du produit du stade précédent et 400 ml de chlorure de méthylène. On agite pendant 1 heure à 0°C, lave avec une solution 1 M de phosphate acide de sodium et rince à l'eau. On sèche. On chromatographie le produit obtenu sur silice en éluant avec le mélange chlorure de méthylène/acétone 99-1 puis avec le mélange toluène/tertbu-tylméthyléther. On obtient 28,37 g du produit recherché.

### STADE C : [7R-(7.alpha.,8.bêta.,9.bêta.,10.alpha.)]-4-(diphénylméthoxy)-7-[[10-méthoxy-8-[(tétrahydro-2H-pyran-2-yl)oxy]-9-[(triéthylsilyl)oxy]-6-oxaspiro[4.5]décan-7-yl)oxy]-8-méthyl-2H-1-benzopyran-2-one

On ajoute 7,57 ml de 3,4-dihydropyran et 400 mg d'acide paratoluène sulfonique dans une solution renfermant 28,1 g du produit du stade précédent et 250 ml de dichlorométhane. On agite 1 heure à la température ambiante - On ajoute du bicarbonate de soude et agite 20 minutes à la température ambiante. On lave à l'eau, sèche les phases organiques sur sulfate de sodium. On chromatographie le produit obtenu sur silice en éluant avec le mélange heptane-acétate d'éthyle 4,1. On obtient 16,81 g de produit recherché.

### STADE D : [7R- (7.alpha.,8.bêta.,9.bêta.,10.alpha.)]-4 hydroxy-7-[[10-méthoxy-8-[(tétrahydro-2H-pyran-2-yl)oxy]-9-[(triéthylsilyl)oxy]-6-oxaspiro[4.5]décan-7-yl)oxy]-8-méthyl-2H-1-benzopyran-2-one On agite sous atmosphère d'hydrogène, une solution de 16,19 g du produit du stade précédent, 150 ml de THF, en présence de 810 mg de palladium sur charbon. On filtre et obtient 15,1 g de produit recherché.

### STADE E : [7R- (7.alpha.,8.bêta., 9.bêta.,10.alpha.,)]-3-acétyl-4-hydroxy-7-[[10-méthoxy-8-[ (tétrahydro-2H-pyran-2-yl)oxy]-9-[(triéthylsilyl)oxy]-6-oxaspiro[4.5]décan-7-yl)oxy]-8-méthyl - 2H-1-benzopyran-2-one

On ajoute 2,28 ml d'anhydride acétique dans un mélange renfermant 13,8 g de produit du stade précédent et 150 ml de chlorure de méthylène et 5,94 g de diméthylaminopyridine (DMAP). On agite une heure à la température ambiante. On traite avec une solution molaire de phosphate acide de sodium, on extrait au chlorure de méthylène, lave à l'eau et sèche . On obtient 16,21 g de produit recherché utilisé tel quel dans le stade suivant.

### STADE F : [7R-(7.alpha.,8.bêta.,9.bêta.,10.alpha.)]-3-acétyl-4-hydroxy-7-[[9-hydroxy-10-méthoxy-8-[(tétrahydro-2H-pyran-2-yl)oxy]-6-oxaspiro[4.5]décan-7-yl)oxy]-8-méthyl-2H-1-benzopyran-2-one

On ajoute à 0°C, 1.5 équivalent d'une solution 1M dans le THF de fluorure de tétrabutylammonium, dans une solution renfermant le produit du stade précédent et 200 ml de THF. On maintient le mélange réactionnel sous agitation à température ambiante pendant 15 heures . On verse le mélange réactionnel sur le mélange heptane-acétate d'éthyle 30-70. On lave à l'eau, filtre et sèche. On obtient un produit que l'on utilise tel quel dans le stade suivant.

### STADE G : [7R-(7.alpha.,8.bêta., 9.bêta-,10.alpha.)] -4-hydroxy-7-[[9-hydroxy-10-méthoxy-8-[(tétrahydro-2H-pyran-2-yl)oxy]-6-oxaspiro[4.5]décan-7-yl)oxyl-3-[1-(méthoxyimino) éthyl]-8-méthyl-2H-1-benzopyran-2-one

On ajoute 4,6 g d'acétate de potassium et 3,12 g de chlorhydrate de O-méthylhydroxylamine dans une solution renfermant 18,69 mmoles de produit du stade précédent et 100 ml d'éthanol. On agite pendant 1,5 heure à la température ambiante. On verse sur une solution 1M de phosphate acide de sodium, extrait avec un mélange heptane/acétate d'éthyle 30-70. On lave à l'eau, sèche et évapore à sec. On chromato-graphie le produit obtenu avec un mélange heptane-acétate d'éthyle (1:1). On obtient 6,54 g de produit recherché.

### STADE H : [7R.(7-alpha.,8.bêta.,9.bêta.,10.alpha.)]-(2-propynyloxy)-carbamate de 8-hydroxy-7-[4-hydroxy 3-[1-(méthoxyimino)éthyl]-8-méthyl-2-oxo-2H-1-benzopyran-7-yl]-10-méthoxy-6-oxaspiro[4.5]décan-9-yle

1) On introduit à 0°C 3,70 g de DMAP et 3,05 g de chloro-formiate de para-nitrobenzène dans une solution renfermant 6,37 g de produit du stade précédent et 70 ml de dichloro-méthane. On agite 1 heure à 0°C.
2) On ajoute 2,3 g d'hydrure de sodium à 0°C dans une solution renfermant 6,26 g de chlorohydrate propargylhy-droxylamine et 50 ml de DMF. On agite 1 heure à 0°C.

La solution (1) est concentrée à sec. Le résidu obtenu est mis en solution dans 50 ml de DMF. On ajoute 1,42 g de DMAP. On ajoute à 0°C la solution (2) dans la solution ainsi obtenue. On agite 1 heure à 0°C. On traite avec du phosphate acide de sodium, lave à l'eau, sèche et concentre à sec. On met le résidu obtenu en solution dans 100 ml de méthanol. On ajoute 2,1 g d'APTS et agite à la température ambiante. On chromatographie le produit obtenu en éluant avec du toluène et puis avec un mélange toluène-éther isopropylique 92-8. On disperse le produit sous ultra-sons dans un mélange éther isopropylique-pentane. On obtient le produit recherché. Spectre RMN : CDCl₃ ppm

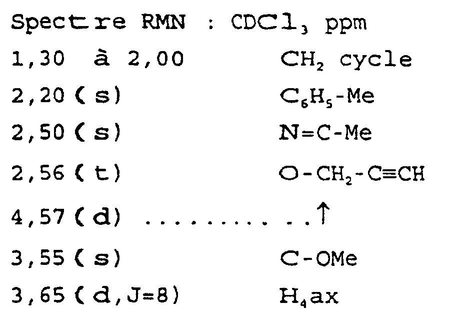

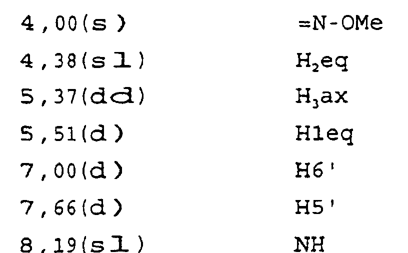

### Préparation 4

### STADE A :

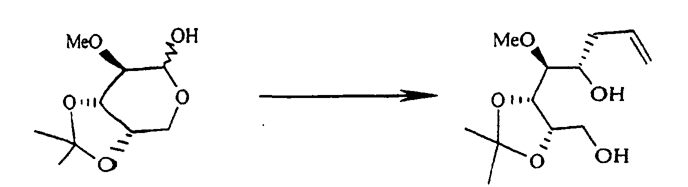

On met en solution sous atmosphère d ' argon, 20,4 g de 2-0-méthyl-3,4-0-(1-méthyléthylédène)L-arabinose dans 200 ml de tétrahydrofurane. On ajoute à 0°C sous argon, 200 ml de solution de bromure d'allylmagnésium 2 M dans le tétrahydrofurane. La solution est agitée 1 heure à 0°C. Le milieu réactionnel est refroidi à -15°C et est dilué avec 100 ml d'heptane. Pour neutraliser l'excès de magnésium, on ajoute goutte à goutte 300 ml d'une solution aqueuse d'hydrogéno-sulfate de sodium à 10%. La phase organique est séparée et la phase aqueuse est extraite avec un mélange d'heptane 1/acétate d'éthyle 2. Les phases organiques sont rassemblées, séchées sur sulfate de magnésium et évaporées à sec. On obtient 22,96 g de produit recherché.
Rendement : 94 %

### STADE B :

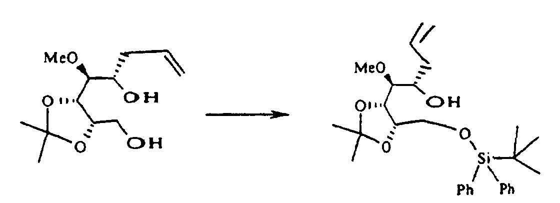

On met en solution sous atmosphère d ' argon, 22,96 g du produit du stade précédent dans 175 ml de diméthylformamide. On ajoute 14,88 g d'imidazole puis à 0°C sous argon, goutte à goutte pendant 30 minutes, 23,31 ml de chlorure de diphénylterbutylsilyle. La solution est agitée 30 minutes à 0°C. Le milieu réactionnel est dilué avec 4 O0 ml de mélange heptane 1/acétate d'éthyle 2. La phase organique est lavée avec deux fois 200 ml d'une solution aqueuse de dihydrogénophosphate de sodium à 1 molaire, séchée sur sulfate de magnésium et évaporée à sec. On obtient 45 g de produit résine qui est purifié par chromatographie sur silice en éluant avec le mélange heptane 4/acétate d'éthyle 1. On obtient : 39,5 g de produit recherché.
Rendement : 85%

### STADE C :

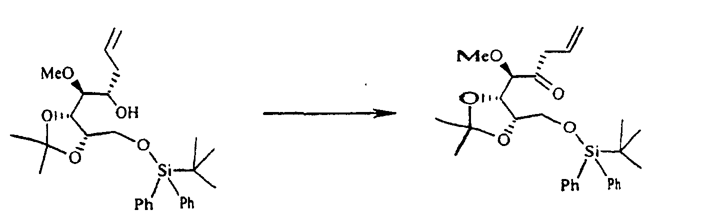

On met en suspension 25,1 g de chlorochromate de pyridinium dans 200 ml de chlorure de méthylène. On ajoute alors 53,8 g de tamis moléculaire 4Å. A cette suspension, on introduit en une seule fois 39,5 g de produit du stade précédent en solution dans 100 ml de chlorure de méthylène. On agite pendant 3 heures. La suspension est filtrée. On élue avec un mélange chlorure méthylène 3% méthanol. Le filtrat est évaporé à sec. Le résidu obtenu (35 g) est filtré sur silice en éluant avec le mélange heptane 4/acétate d'éthyle 1. On obtient 32,9 g de produit recherché.
Rendement : 87%

### STADE D :

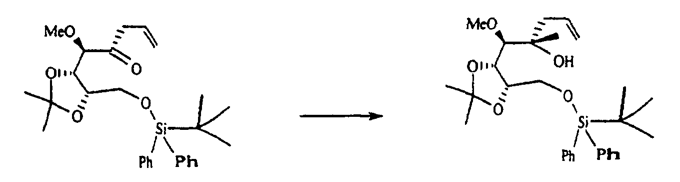

On met en solution 32,5 g de produit du stade précédent dans 250 ml de tétrahydrofurane. Sous argon, à -5°C, on ajoute goutte à goutte, 60 ml de solution de bromure de méthylmagnésien dans l'éther (3M). On agite pendant 1 heure à la température ambiante. A 0°C, on neutralise l'excès de magnésien avec une solution aqueuse d'hydrogénosulfate de sodium à 10%. On ajoute 200 ml d'un mélange d'heptane l/acétate d'éthyle 2. La phase organique est lavée avec 200 ml d'une solution aqueuse de dihydrogénophosphate de sodium (M) , séchée sur sulfate de magnésium et évaporée à sec. On empâte dans 200 ml de pentane/éther le produit obtenu. On obtient 16,9 g de produit recherché.
Rendement : 64%

### STADE E :

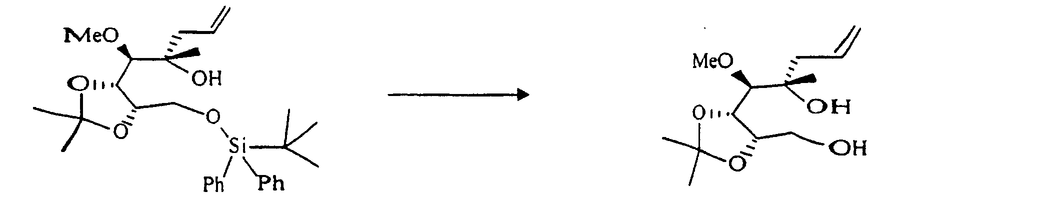

On met en solution 16, 9 g de produit du stade précédent dans 150 ml de tétrahydrofurane. Sous argon, à 0°C, on ajoute goutte à goutte, 68 ml de solution molaire de fluorure de tétrabutylammonium dans le tétrahydrofurane. On agite pendant 30 minutes à température ambiante. On ajoute 200 ml d'un mélange d'heptane 1/acétate d'éthyle 2. La phase organique est lavée avec 200 ml d'une solution aqueuse de dihydrogénophosphate de sodium molaire, séchée sur sulfate de magnésium et évaporée à sec. Le produit brut est purifié par chromatographie sur silice en éluant avec le mélange chlorure de méthylène 95/méthanol.5. On obtient 10,1 g de produit recherché.

### STADE F :

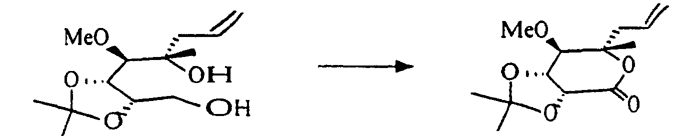

On met en solution 10, 15 g de produit du stade précédent dans 103 ml de chlorure de méthylène. Sous argon, à température ambiante, on ajoute 55ml de triéthylamine et 103 ml de diméthylsulfoxyde stocké sur tamis moléculaire. La solution est refroidie à environ 5°C avec un bain de glace-eau et on ajoute par fraction, 19,77 g de pyridine sulfur-trioxyde sans que la température ne dépasse 15°C. On agite pendant 1 heure. Le milieu réactionnel est versé dans 1 litre de solution aqueuse de dihydrogénophosphate de sodium molaire, la phase aqueuse est extraite deux fois avec un mélange heptane 1/acétate d'éthyle 2. La phase organique est lavée à l'eau, séchée sur sulfate de magnésium et évaporée à sec. Le produit brut cristallise et est empâté dans du pentane. On obtient 6,8 g de produit recherché .
Rendement : 68%

### STADE G :

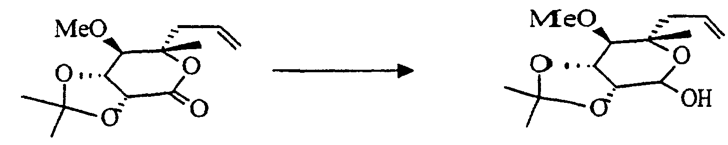

On met en solution 5,3 g de produit du stade précédent dans 30 ml de tétrahydrofurane. Sous argon, à -6°C, on ajoute 13,85 ml. de DIBAL. Après 1 heure 30 d'agitation à 0°C, la réaction est terminée. Le milieu réactionnel est versé dans 100 ml de solution à 1M de tartrate double de sodium et potassium ; la phase aqueuse est extraite avec un mélange heptane 1/acétate d'éthyle 2. La phase organique est lavée avec 150 ml de solution aqueuse d'hydrogénosulfate de sodium à 10%, séchée sur sulfate de magnésium et évaporée à sec. On obtient 5,5 g de produit recherché.
Rendement : Quantitatif

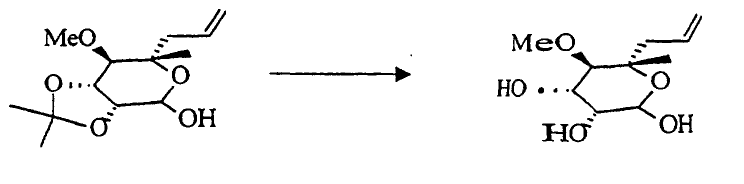

### STADE H :

On met en émulsion 5,5 g de produit du stade précédent dans 32 ml de solution d'acide sulfurique à 0,05 N. Après 1 heure 30 de chauffage à 70°C, la réaction est terminée. On laisse revenir à température ambiante et le milieu réactionne 1 est neutralisé avec 0,6 g de carbonate de baryum. La suspension est agitée une heure à température ambiante (pH=7), puis filtrée et évaporée à sec. Pour sécher le produit, on fait deux entraînements au toluène. On sèche et obtient 4,4 g de produit recherché.
Rendement : 96%

### EXEMPLE 6 :

### 7-[[6-déoxy-4-O-méthyl-5-C-(2-propényl)-3-0-[[(2-propynyloxy)amino]carbonyl]-.bêta-D-gulopyranosyl]oxy]-4-hydroxy-8-méthyl-3-[1- [(2-propynyloxy)imino]éthyl]-2H-1-benzopyran-2-one et

### 7-[[6-déoxy-4-O-méthyl-5-C- (2-propényl)-3-0-[[(2-propynyloxy)amino]carbonyl]-.bêta.-D-gulopyranosyl]oxy]-4-hydroxy-3-[1-(méthoxyimino)éthyl]-8-méthyl-2H-1-benzopyran-2-one

### STADE A :

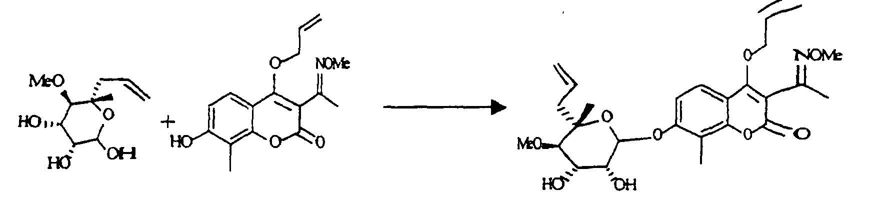

On met en solution 4,4 g du produit de la préparation 4 en solution dans 100 ml de chlorure de méthylène. A température ambiante, sous argon, on ajoute 7,33 g de coumarine (7-hydroxy-3- [ (méthoxyimino)méthyl]-8 -méthyl -4 - (2-propényloxy)-2H-1-benzopyran-2-one) préparée comme indiqué à la préparation 8 de la demande de brevet international WO 9747634 et 6,29 g de triphénylphosphine. La suspension est refroidie à 0°C. On ajoute goutte à goutte 3.73 ml de DEAD. La suspension est agitée 1 heure à température ambiante. On ajoute de nouveau 6,06 g de triphénylphosphine et à 0° C 3,11 ml de DEAD. Après 1 heure d'agitation à température ambiante, on ajoute 50 ml de pentane pour faire précipiter le DEAD réduit. La suspension est filtrée, le filtrat est évaporé à sec et purifié sur silice avec le mélange éluant toluène à 3% puis 6 % d'alcool isopropylique. On obtient 7,1 g de produit. Le produit est filtré sur silice 60 en éluant avec un mélange éther/heptane puis à l'éther. On obtient 6,13 g de produit recherché.

### STADE B :

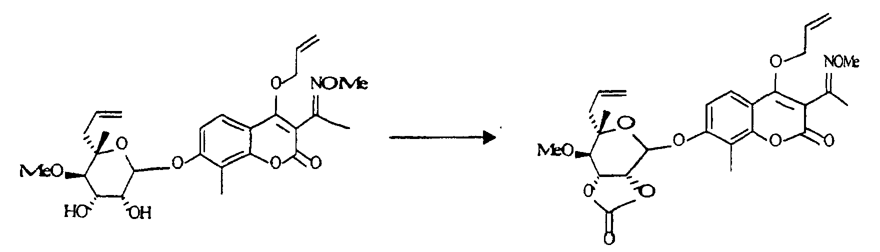

On met en solution 6 g de produit du stade précédent dans 75 ml de tétrahydrofurane. On ajoute 3,86 g de carbonyldi-imida zole et la réaction est chauffée 1 heure au reflux. Le milieu réactionnel est dilué avec 100 ml de mélange heptane 1/acétate d'éthyle 2. La phase organique est lavée avec une solution aqueuse d'hydrogénosulfate de sodium à 10 %, séchée sur sulfate de magnésium et évaporée à sec.
On obtient 4,94 g de produit recherché.

### STADE C :

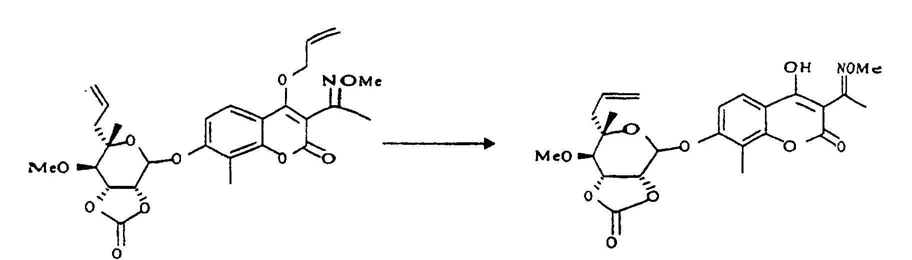

On met en solution 4,94 g de produit du stade précédent dans 120 ml de tétrahydrofurane. On ajoute 8,44 ml de diisopropylamine à 0°C, 1,05 g de palladium tétrakistriphényl-phosphine. On agite pendant 20 minutes à 0°C. Le milieu réactionnel est dilué avec 50 ml de mélange heptane 1/acétate d'éthyle 2. La phase organique est lavée avec une solution aqueuse d'hydrogénosulfate de sodium à 10%, séchée sur sulfate de magnésium et évaporée à sec. On obtient 5, 5 g de produit brut qui est purifié sur silice en éluant avec le mélange chlorure de méthylène à 2% d'acétone.
On obtient 3,1 g de produit recherché.

### STADE D :

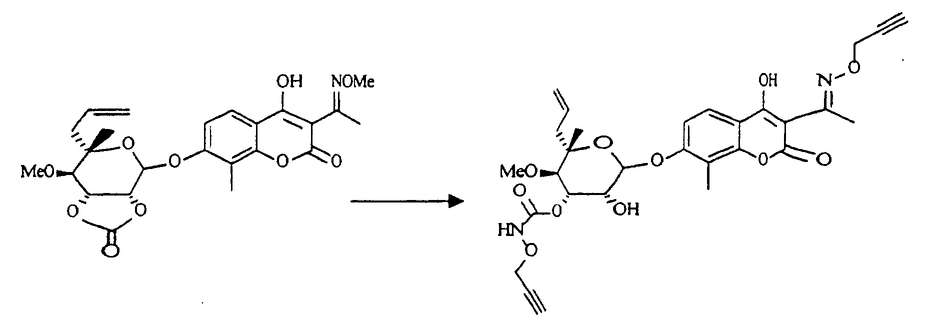

On met en solution 0,65 g de produit du stade précédent dans 6,5 ml de pyridine séchée sur potasse. A température ambiante, on ajoute 1,5 g du chlorhydrate de la propargylhydroxylamine et 0,149 de perchlorate de lithium. On agite à la température ambiante pendant 48 heures. On dilue avec un mélange heptanel/acétate d'éthyle 2 et la phase organique est lavée avec une solution d'hydrogénosulfate de sodium à 10%, séchée sur sulfate de magnésium. On obtient 1,8 g de produit que l'on purifie par chromatographie sur silice en éluant avec le mélange éluant chlorure de méthylène 80/terbutylméthylether 20.
On obtient 200 mg de produit recherché isomère en 3 et 500 mg de l'isomère en 2.

### STADE E :

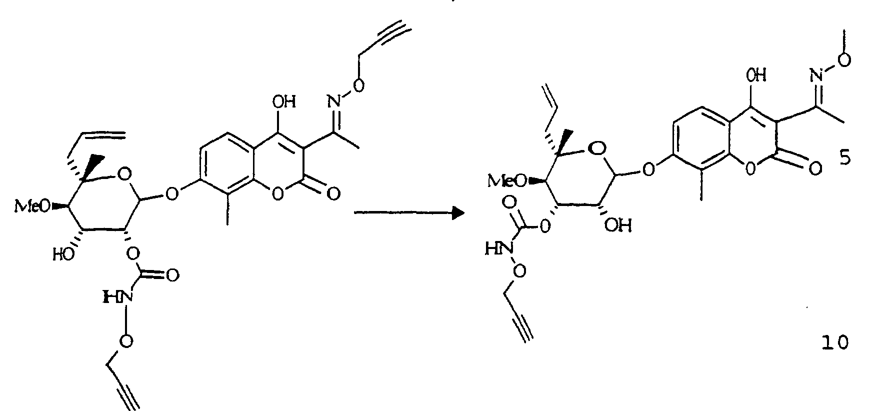

On met en solution sous atmosphère d'argon 0,5 g de l'isomère en 2 obtenu au stade précédent dans 10 ml de chlorure de méthylène. On ajoute 100 µl de DBU. On agite 24 heures à température ambiante. On dilue dans 50 ml de mélange heptane l/acétate d'éthyle 3 et lave la phase organique avec une solution de dihydrogénosulfate de sodium à 1 M, sèche sur sulfate de magnésium et évapore à sec. Le produit précédemment obtenu est mis en solution dans 5 ml d'éthanol. A température ambiante, on ajoute 0,72 g de chlorhydrate de méthylhydroxylamine et 0,94 g d'acétate de sodium. Le milieu réactionnel est agité 5 heures à température ambiante. On dilue dans 50 ml de mélange heptane 1/acétate d'éthyle 3 et lave la phase organique avec une solution de dihydrogénosulfate de sodium à 1 M, sèche sur sulfate de magnésium et évapore à sec. On obtient 0 , 45 g de produit brut qui est purifié par chromatographie sur silice avec le mélange éluant chlorure de méthylène 80/20 terbutylméthylether 20.
On obtient 100 mg de produit recherché.

### Préparation 5

### STADE A :

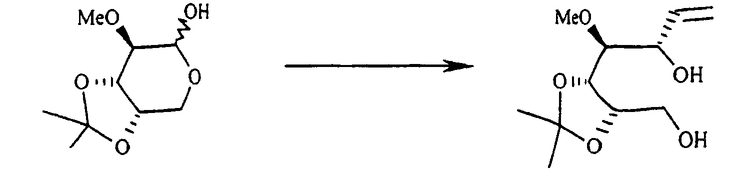

On met en solution sous argon 20,4 g de 2-0-méthyl-3,4-0(1-méthyléthylidène)L-arabinose dans 250 ml de tétrahydrofurane. On ajoute à 0°C sous argon, 100 ml de solution de bromure de vinylmagnésium, 1 M dans le tétrahydrofurane puis 200 ml de solution de chlorure de magnésium, 1.7 M dans le tétrahydrofurane ; 0,34 moles) . La solution est agitée 1 heure à température ambiante. Le milieu réactionnel est refroidi à -15°C et est dilué avec 100 ml d'heptane. Pour neutraliser l'excès de magnésien, on ajoute goutte à goutte 300 ml d'un mélange à 20% d'une solution, aqueuse de dihydrogénophosphate de sodium molaire dans le tétrahydrofurane. Les sels de magnésium précipitent. On ajoute 200 ml de
mélange heptane 1/acétate d'éthyle 2 et 150 ml d'une solution à 10% d'hydrogénosulfate de sodium. La solution organique est séchée sur sulfate de magnésium et évaporée à sec. On obtient 19,3 g de produit recherché.
Rendement : 83%

### STADE B :

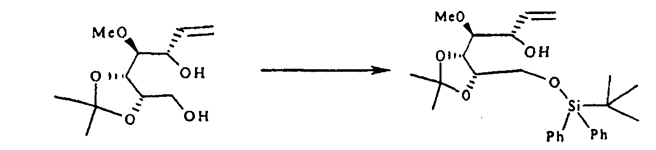

On met en solution 19,3 g de produit du stade précédent dans 150 ml de diméthylformamide. On ajoute 10,8 g d'imidazole puis à 0°C sous argon, goutte à goutte pendant 30 minutes 23,4 ml de chlorure de diphénylterbutyls ilyle. La solution est agitée 30 minutes à 0°C . Le milieu réactionnel est dilué avec 400 ml de mélange heptane 1/acétate d'éthyle 2. La phase organique est lavée avec une solution aqueuse de dihydrogénophosphate de sodium 1 M séchée sur sulfate de magnésium et évaporée à sec. On obtient 30,2 g de produit résine qui est purifié par chromatographie sur silice en éluant avec le mélange heptane 4 : acétate d'éthyle 1. On obtient 30,2 g de produit recherché. Rendement : 77%

### STADE C :

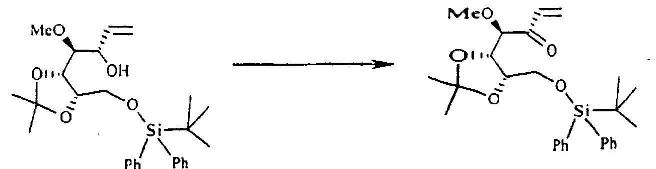

On met en solution 19,1 g de chlorochromate de pyridinium dans 250 ml de chlorure de méthylène . On ajoute alors 40 g de tamis moléculaire 4Å. A cette suspension, on introduit en une seule fois 28,19 g de produit du stade précédent en solution dans 100 ml de chlorure de méthylène. Après 4 heures d'agitation à température ambiante, la réaction est finie. On filtre. Le filtrat est évaporé à sec. On chromatographie le produit obtenu sur silice en éluant avec le mélange heptane/acétate d'éthyle 6-1. On obtient 10,5 g de produit recherché. Rendement 36 %.

### STADE D :

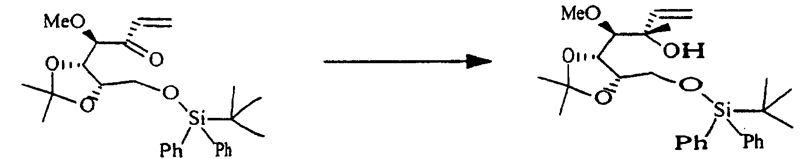

On met en solution 10 g de produit du stade précédent dans 100 ml de tétrahydrofurane. Sous argon, à -5°C, on ajoute goutte à goutte, 14 ml de solution de bromure de méthylmagnésien dans l'éther 3M. On agite 30 minutes à O°C, neutralise l'excès de magnésien avec une solution aqueuse de hydrogénosufate de sodium à 10 %. On ajoute 200 ml de mélange heptane 1/acétate d'éthyle 2. La phase organique est lavée avec 200 ml d'une solution aqueuse de dihydrogéno-phosphate de sodium molaire, séchée sur sulfate de magnésium et évaporée à sec. Le produit brut est purifié sur silice en éluant avec le mélange heptane 4/acétate d'éthyle 1. On empâte dans le pentane le produit obtenu. On obtient 2,76 g de produit recherché.
Rendement 27%

### STADE E :

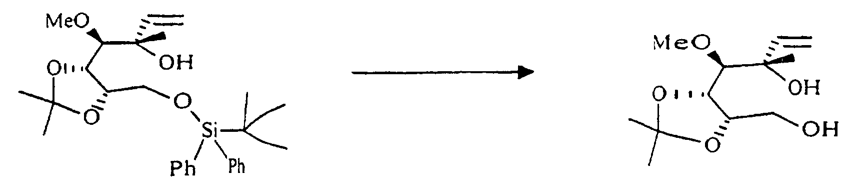

On met en solution 2,79 g de produit du stade précédent dans 15 ml de tétrahydrofurane. Sous argon, à 0°C, on ajoute goutte à goutte, 11,8 ml de solution molaire de fluorure de tétrabutylammonium dans le tétrahydrofurane. On agite 1 heure à la température ambiante. On ajoute 200 ml d'un mélange d'heptane 1/acétate d'éthyle 2. La phase organique est lavée avec 200 ml d'une solution aqueuse de dihydrogénophosphate de sodium molaire, séchée sur sulfate de magnésium et évaporée à sec. Le produit brut est purifié par chromatographie de silice en éluant avec le mélange chlorure de méthylène à 5% méthanol. On obtient 1,2 g de produit recherché. Rendement : 8 6%

### STADE F :

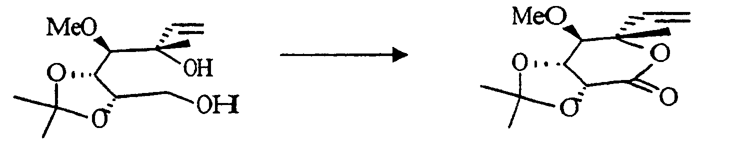

On met en solution 1,2 g de produit du stade précédent dans 12,5 ml de chlorure de méthylène. Sous argon, à température ambiante, on ajoute 6,67 ml de triéthylamine et 12,5 ml de diméthylsulfoxide stocké sur tamis moléculaire. La solution est refroidie à environ 5°C avec un bain de glace- eau et on ajoute par fraction 2,39 g de pyridine sulfurtrioxide sans que la température ne dépasse 15°C. Après 1 heure d'agitation à température ambiante, la réaction est terminée. On agite 1 heure à température ambiante. Le milieu réactionnel est versé dans 100 ml de solution aqueuse de dihydrogénophosphate de sodium molaire, la phase aqueuse est extraite deux fois avec un mélange heptane 1/acétate d'éthyle 2. La phase organique est lavée à l'eau, séchée sur sulfate de magnésium et évaporée à sec. On empâte le produit obtenu dans du pentane. On obtient 0,75 g de produit recherché .
Rendement : 59%

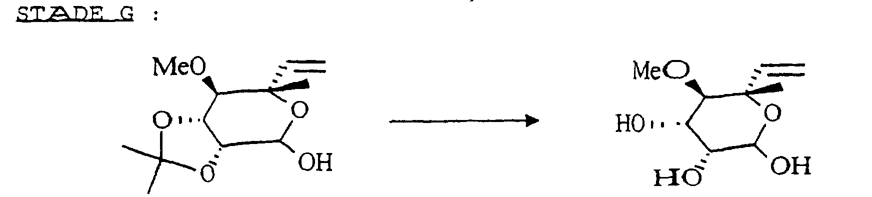

On met en solution 0,73 g du produit du stade précédent dans 30 ml de tétrahydrofurane. Sous argon, à -6°C, on ajoute 2,5 ml de solution à 1,5 M de DIBAL dans le toluène On agite 1 heure 30 à -6°C. Le milieu réactionnel est versé dans une solution à 1M de tart rate double de sodium et potassium ; la phase aqueuse est extraite avec un mélange heptane 1/acétate d'éthyle 2 . La phase organique est lavée avec une solution aqueuse d'hydrogénosulfate de sodium à 10%, séchée sur sulfate de magnésium et évaporée à sec. On empâte le produit obtenu dans du pentane. On obtient 0,95 g de produit recherché. Rendement quantitatif.

### STADE H :

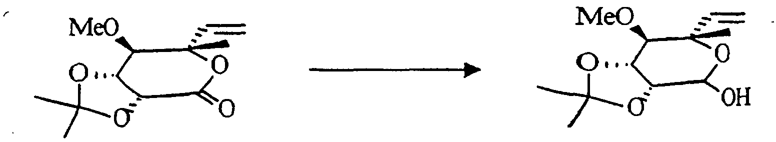

On met en émulsion 0,9 g de produit du stade précédent dans 5 ml de solution d'acide sulfurique à 0,05 N. Après 1 heure de chauffage à 70°C, la réaction est terminée. On laisse revenir à température ambiante ; le milieu réactionnel est extrait au pentane et la phase aqueuse est neutralisée avec 0,1 g de carbonate de baryum ( La suspension est agitée une heure à température ambiante ( pH=7) , puis filtrée et évaporée à sec. Pour sécher le produit, on fait deux entraînements au toluène, puis on solubilise dans du chlorure de méthylène, sèche la solution sur sulfate de magnésium et évapore à sec. On obtient 0,5 g de produit recherché.
Rendement 86%.

### EXEMPLE 7 : 7- [[6-déoxy-5-C-éthényl-4-O-méthyl-3-O-[[(2-propynyloxy)amino]carbonyl]-.bêta.-D-gulopyranosyl]oxy]-4-hydroxy-3-[1-(méthoxymino)éthyl]-8-méthyl-2H-1-benzopyzan-2- one

### STADE A :

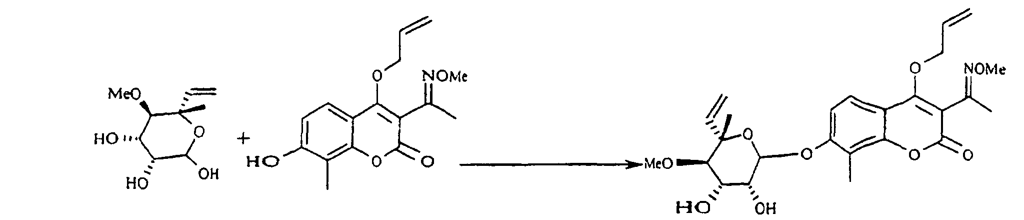

On met en solution 0,5 g de produit de la préparation 5 dans 17 ml de chlorure de méthylène. A température ambiante, sous argon, on ajoute 0,89 g de coumarine préparée comme indiqué dans la demande de brevet international WO9747634 et 0, 76 g de triphénylphosphine. La suspension est refroidie à 0°C, on ajoute goutte à goutte 0,45 ml de DEAD. La suspension est agitée 1 heure à température ambiante. On a j oute de nouveau 0,63 g de triphénylphosphine et à 0°C, 0,37 ml de DEAD. On obtient une solution jaune. Après 1 heure d'agitation à température ambiante, on ajoute 10 ml de pentane pour faire précipiter le DEAD réduit. La suspension est filtrée, le filtrat est évaporé à sec et purifié par chromatographie sur silice avec le mélange éluant toluène 97/alcool isopropylique 3 (on finit l'élution avec 6%). Le produit obtenu en mélange est ensuite filtré sur silice 60 en éluant avec le mélange heptane 1/éther 2 puis éther. On obtient 0,55 g de cristaux blancs. Rendement : 47%.

### STADE B :

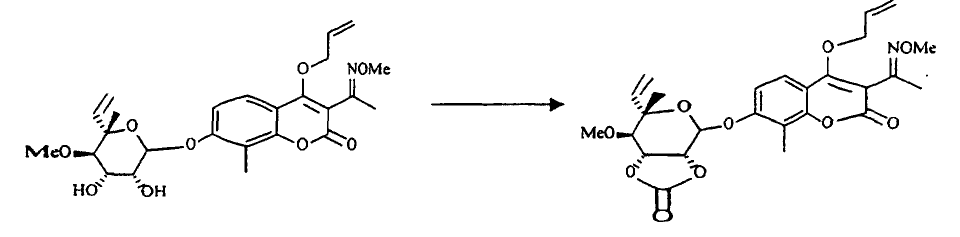

On met en solution 0,55 g de produit du stade précédent dans 7 ml de tétrahydrofurane. On ajoute 0,364 g de carbonyl-diimidazole et le mélange réactionnel est chauffé 1 heure au reflux Le milieu réactionnel est dilué avec 40 ml de mélange heptane 1/acétate d'éthyle 2.La phase organique est lavée avec 50 ml d'une solution aqueuse d'hydrogéno-sulfate de sodium à 10%, séchée sur sulfate de magnésium et évaporée à sec. On obtient 0,5 g de produit recherché.
Rendement : 88%

### STADE C :

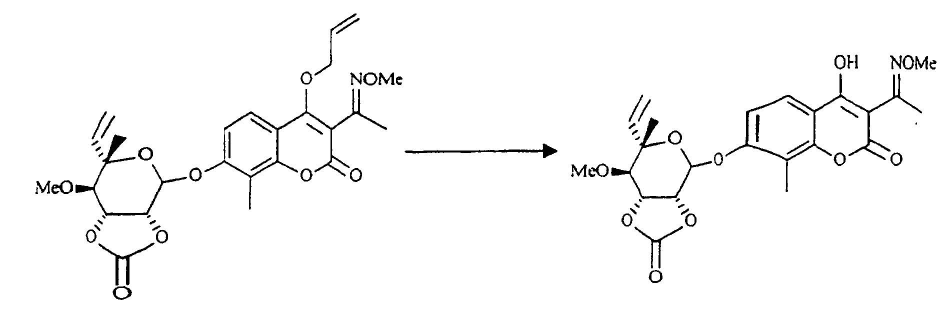

On met en solution 0,5 g de produit du stade précédent dans 12 ml de tétrahydrofurane. On ajoute 0,82 ml de di isopropylamine et à 0°C, 0,11 g de palladium tétrakistriphénylphosphine (0,1 équivalent). On agite pendant 20 minutes à 0°C. Le milieu réactionnel est dilué avec 50 ml de mélange heptane l/acétate d'éthyle 2. La phase organique est lavée avec 50 ml d'une solution aqueuse d'hydrogénosulfate de sodium à 10%, séchée sur sulfate de magnésium et évaporée à sec. On obtient 0,58 g de produit brut qui est purifié par chromatographie sur silice en éluant avec le mélange heptane 3/acétate d'éthyle 1. On obtient 0,257 g de produit recherché.
Rendement : 57%.

### STADE D :

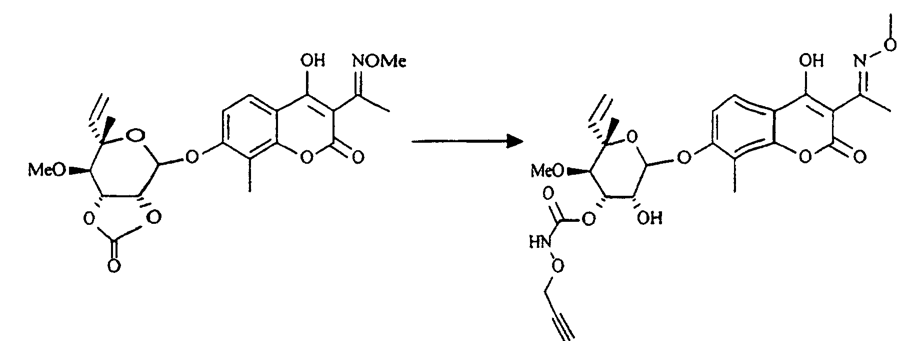

On met en solution 0,257 g de produit du stade précédent dans 2,5 ml de pyridine séchée sur potasse. A température ambiante, on ajoute 0,58 g du chlorhydrate de la propargyl-hydroxylamine et 0,057 g de perchlorate de lithium. Le milieu réactionnel est agité 48 heures à température ambiante. On dilue avec un mélange heptane 1/acétate d'éthyle 2 et la phase organique est lavée avec une solution d'hydrogénosulfate de sodium à 10%, séchée sur sulfate de magnésium. On obtient 0 , 28 g de produit recherché. Le produit brut obtenu est mis en solution dans 5 ml d'éthanol, on ajoute 0,45 g de chlorhydrate de méthylhydroxylamine et 0,58 g d'acétate de sodium. Le milieu réactionnel est agité 5 heures à température ambiante. On dilue avec un mélange heptane 1/acétate d'éthyle 2 et la phase organique est lavée avec une solution dihydrogénophophate de sodium (1 M), séchée sur sulfate de magnésium et évaporée à sec. On obtient 0,3 g de produit brut qui est purifié par chroma-tographie sur silice en éluant avec le mélange chlorure de méthylène 80/acétate d'éthyle 19/acide acétique 1. On obtient 0,090 g de produit recherché. Rendement : 31%.

### Préparation 6

### STADE A :

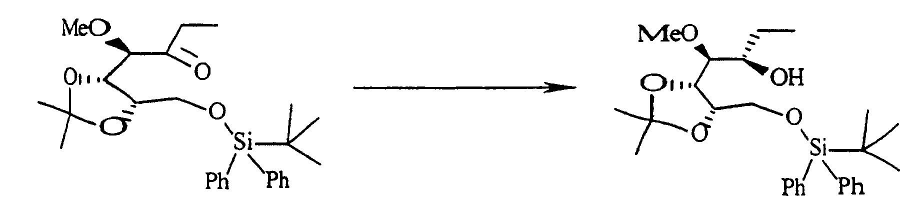

On met en solution 10,5 g dans 110 ml de tétrahydrofurane. Sous argon, à -6°C, on ajoute 329 ml de solution de tétraborohydrure de zinc à 0,135 M dans l'éther. On laisse agiter 30 minutes sans bain de glace, la réaction est alors terminée. On ajoute une solution de dihydrogénophosphate de sodium (M) . La phase aqueuse est extraite avec un mélange heptane 1/acétate d'éthyle 2 .La phase organique est séchée sur sulfate de magnésium et évaporée à sec. On obtient 10,5 g de produit recherché qui est purifié par chromatographie en éluant avec le mélange heptane 4/acétate d'éthyle 1. On obtient 8, 75 g de produit recherché. Rendement : 83%

### STADE B :

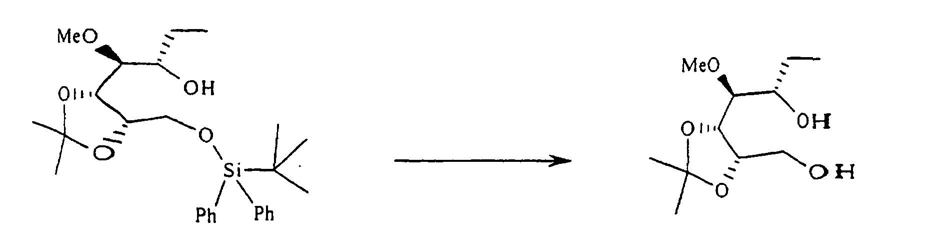

On met en solution 8,75 g du produit du stade précédent dans 100 ml de tétrahydrofurane. Sous argon, à 0°C, on ajoute 37 ml de solution molaire de fluorure de tétrabuthylammonium dans le tétrahydrofurane. Après 30 minutes d'agitation à 0°C, on ajoute 200 ml d'un mélange d'heptane 1/acétate d'éthyle 2. La phase organique est lavée avec 200 ml d'une solution aqueuse de dihydrogénophosphate de sodium molaire, séchée sur sulfate de magnésium et évaporée à sec. Le produit brut (10, 5 g) est purifié par chromatographie en éluant sur silice le mélange chlorure de méthylène à 20% d'acétone. On obtient 3,6 g de produit recherché.
Rendement : 78%.

### STADE C :

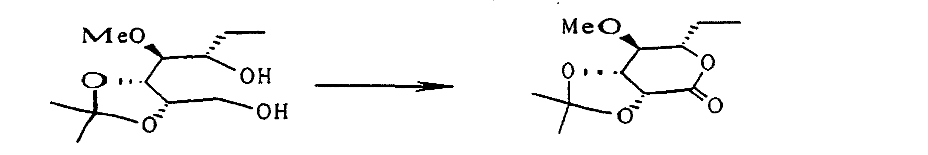

On met 3,57 g de produit du stade précédent dans 38 ml de chlorure de méthylène. Sous argon, à température ambiante, on ajoute 20,5 ml de triéthylamine et 38 ml de diméthylsulfoxide. La solution est refroidie à environ 5°C et on ajoute 7,6 g de pyridine sulfur trioxide sans que la température ne dépasse 15°C. On agite pendant 2 heures. Le milieu réactionnel est versé dans 500 ml de solution aqueuse de dihydrogénophosphate de sodium molaire, la phase aqueuse est extraite deux fois avec un mélange heptane 1/acétate d'éthyle 2 .La phase organique est lavée avec deux fois 500 ml d'eau, séchée sur sulfate de magnésium et évaporée à sec. Le produit brut cristallise et est empâté dans du pentane. On obtient 1,92 g de produit recherché. Rendement : 56%

### STADE D :

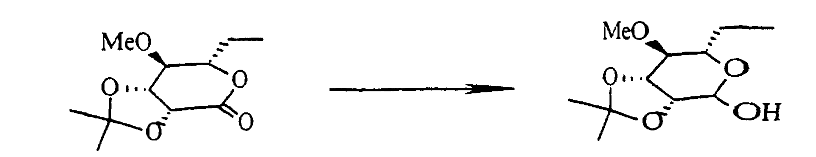

On met en solution 1,9 g de produit du stade précédent dans 10 ml de tétrahydrofurane. Sous argon, à 0°C, on ajoute 6,66 ml de solution à 1. 5M de DIBAL dans le toluène. On agite pendant 1 heure 30. Le milieu réactionnel est versé dans 100 ml de solution à 1M de tartrate double de sodium et potassium ; la phase aqueuse est extraite avec un mélange heptane l/acétate d'éthyle 2. La phase organique est lavée avec 150 ml de solution aqueuse d'hydrogénosulfate de sodium à 10%, séchée sur sulfate de magnésium et évaporée à sec. On obtient 1,9 g de produit recherché.
Rendement : Quantitatif

### STADE E :

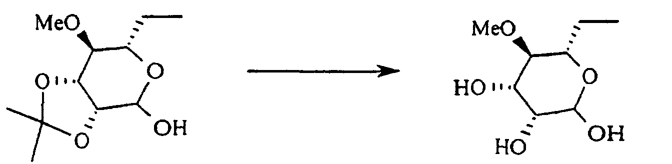

On met en émulsion 1,95 g de produit du stade précédent dans 11,5 ml de solution d'acide sulfurique à 0,05 N. On chauffe pendant 1 h 30 à 70°C. On laisse revenir à température ambiante et le milieu réactionnel est neutralisé avec 0,3 g de carbonate de baryum. La suspension est agitée une heure à température ambiante (pH=7), puis filtrée et évaporée à sec. Pour sécher le produit, on fait deux entraînements au toluène. Après séchage ( une nuit à 40°C en présence de P₂O₅), on obtient 1,2 g de produit recherché.
Rendement : Quantitatif

### EXEMPLE 8 :

### 7- [ (6-déoxy-6-C-méthyl-4-O-méthyl-3-O-[[(2-propynyloxy)-amino]carbonyl]-.alpha.-L-mannopyranosyl)oxy]-4-hydroxy-8- méthyl-3-[1-[(2-propynyloxy)imino]éthyl]-2H-1-benzopyran-3-yl]-2-one

### 7- [(6-déoxy-6-C-méthyl-4-O-méthyl-3-O-[[(2-propynyloxy)-amino]carbonyl]-.alpha.-L-mannopyranosyl)oxy]-4-hydroxy-3-[1-(méthoxyimino)éthyl]-8-méthyl-2H-1-benzopyran-3-yl]2-one

### STADE A :

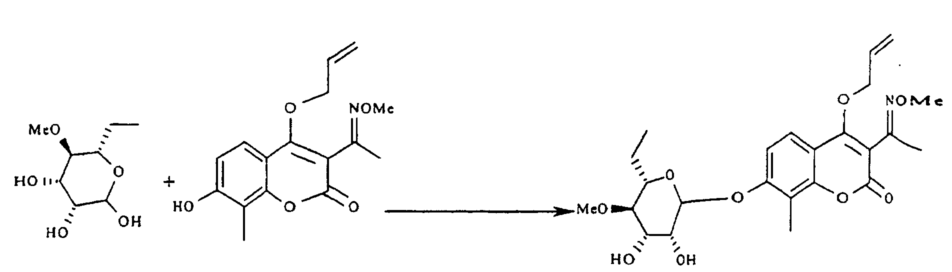

On met en solution 1,16 g du produit de la préparation 6 dans 25 ml de chlorure de méthylène. A température ambiante, sous argon, on ajoute 2,19 g de coumarine 7-hydroxy-3 [(méthoxyimino)méthyl]-8-méthyl-4-(2-propényloxy)-2H-1-benzopyran-2-one préparé comme indiqué dans la demande dé brevet international WO9747634 et 1, 89 g de triphényl-phosphine. La suspension est refroidie à 0°C, on ajoute goutte à goutte 1,12 ml de DEAD. La suspension est agitée 1 heure à température ambiante. On ajoute de nouveau 1,58 g de triphénylphosphine et à 0°C 0,93 ml de DEAD. Après 1 heure d'agitation à température ambiante, on ajoute 50 ml de pentane pour faire précipiter le DEAD réduit. La suspension est filtrée, le filtrat est évaporé à sec et purifié par chromatographie sur silice en éluant avec le mélange toluène à 3% d'alcool isopropylique. On obtient 0, 870 g de cristaux blancs et 0,850 g de mélange contenant des traces de DEAD réduit. Le produit est filtré rapidement sur 100 g de silice 60 en éluant à l'éther. On obtient 0,4 g de produit recherché.
Poids total : 1,27 g. Rendement : 44%

### STADE B :

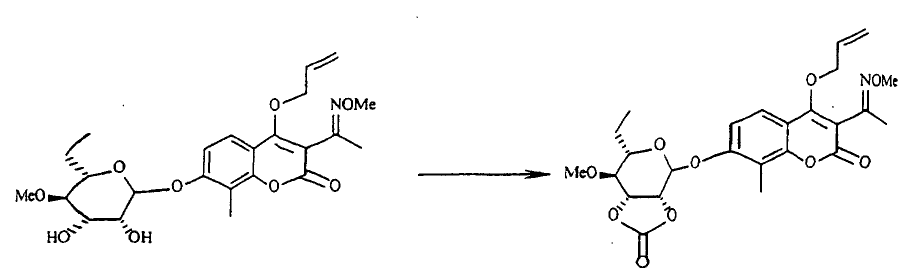

On met en solution 1,27 g du produit du stade précédent dans 10 ml de tétrahydrofurane. On ajoute 0,85 g de carbonyldi-imidazole et chauffe 1 heure au reflux. Le milieu réactionnel est dilué avec 50 ml de mélange heptane 1/acétate d'éthyle 2. La phase organique est lavée avec deux fois 50 ml d'une solution aqueuse d' hydrogénosulfate de sodium à 10%, séchée sur sulfate de magnésium et évaporée à sec. On obtient 1,41 g de produit recherché.
Rendement : Quantitatif

### STADE C :

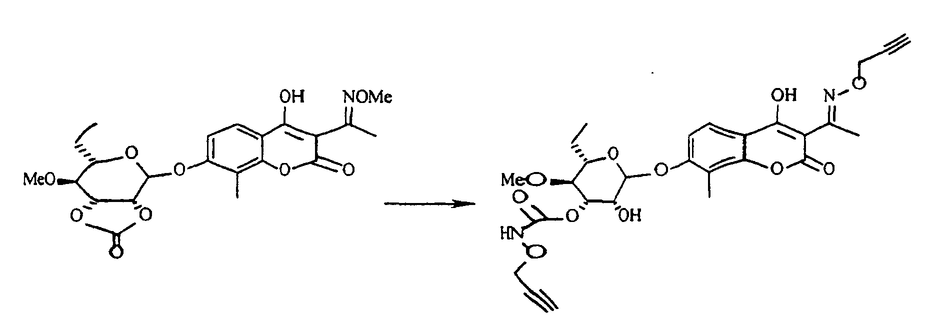

On met en solution 0,6 g de produit du stade précédent dans 6,5 ml de pyridine séchée sur potasse. A température ambiante, on ajoute 1, 5 g du chlorhydrate de la propargyl-hydroxylamine et 0,149 de perchlorate de lithium. On agite pendant 48 heures à température ambiante. On dilue avec un mélange heptane 1/acétate d'éthyle 2 et la phase organique est lavée avec une solution d'hydrogénosulfate de sodium à 10%, séchée sur sulfate de magnésium. On obtient 1,8 g de produit que l'on chromatographie sur silice en éluant avec le mélange chlorure de méthylène 80/acétate d'éthyle 19/acide acétique 1 . On obtient 186 mg de produit recherché isomère en 3, 400 mg isomère en 2.
Rendement : 74% Ouverture du carbonate dont 30% isomère en 3.

### STADE D :

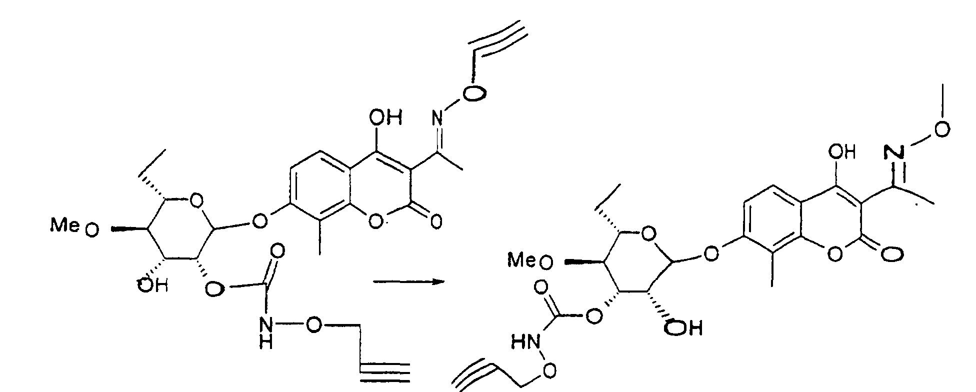

On met en solution 0,4 g de produit du stade précédent (isomère en 2) dans 10 ml de chlorure de méthylène. On ajoute 100 µl de DBU. On agite pendant 24 heures à la température ambiante. On dilue avec un mélange heptane 1/acétate d'éthyle 2 et la phase organique est lavée avec une solution de dihydrogéno-phosphate de sodium à 1 M, séchée sur sulfate de magnésium et évaporée à sec. Dans un ballon de 100 ml, 0,4 g du mélange obtenu précédemment est mis en solution dans 10 ml d'éthanol. A température ambiante , on ajoute 0,59 g de chlorhydrate de méthylhydroxylamine et 0,76 g d'acétate de sodium. Le milieu réactionnel est agité 5 heures à température ambiante. On dilue avec un mélange heptane 1/acétate d'éthyle 2 et la phase organique est lavée avec une solution de dihydrogénophosphate de sodium à 1 M, séchée sur sulfate de magnésium et évaporée à sec. On obtient 0,45 g de produit brut qui est purifié sur silice avec le mélange éluant chlorure de méthylène 80/acétate d'éthyle 19 / acide acétique 1. On isole uniquement l'isomère en 3 attendu. On obtient 0,140 g de produit recherché.
Rendement : 37%

### Préparation 7

### STADE A :

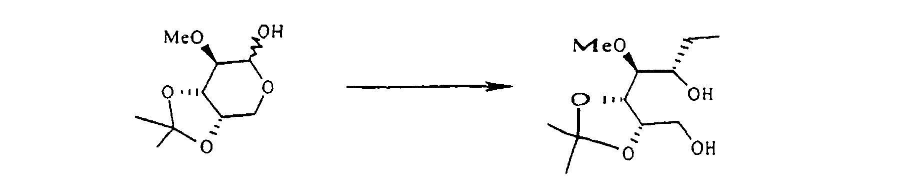

On met en solution 26,8 g de produit sous argon dans 250 ml de tétrahydrofurane. On ajoute à 0°C sous argon, goutte à goutte, 400 ml de solution de bromure d' éthylmagnésium 1 M dans le tétrahydrofurane. La solution est agitée 2 heures à température ambiante. Le milieu réactionnel est refroidie à 0°C et est dilué avec 100 ml d'heptane. Pour neutraliser l'excès de magnésien, on ajoute goutte à goutte 300 ml d'une solution aqueuse de dihydrogénophosphate de sodium molaire. Les sels de magnésium précipitent. On ajoute 200 ml d'un mélange d'heptane 1/acétate d'éthyle 2 et 150 ml d' une solution à 10% d'hydrogénosulfate de sodium. La solution organique est séchée sur sulfate de magnésium et évaporée à sec.
On obtient 29 g de produit qui est chromatographié sur silice en éluant avec le mélange Heptane 1/acétate d'éthyle 4. On obtient 17 g de produit recherché. Rendement 52%.

### STADE B :

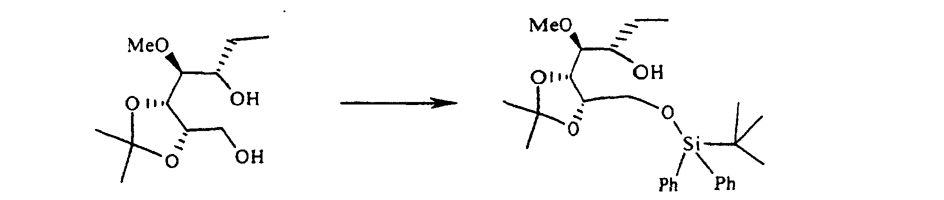

On met en solution 16,7 g de produit du stade précédent sous argon dans 150 ml de diméthylformamide. On ajoute 10,07 g d'imidazole puis à 0°C sous argon, goutte à goutte pendant 30 minutes, 19,23 ml de chlorure de diphénylterbutylsilyle La solution est agitée 1H30 à température ambiante. Le milieu réactionnel est dilué avec 400 ml de mélange heptane 1/acétate d'éthyle 2. La phase organique est lavée avec une solution aqueuse de dihydrogénophosphate de sodium molaire, séchée sur sulfate de magnésium et évaporée à sec. On obtient 38 g de produit qui est purifié par chromatographie sur silice en éluant avec le mélange chlorure de méthylène à 10% d'acétone. On obtient : 33,23 g de produit recherché.
Rendement : Quantitatif

### STADE C :

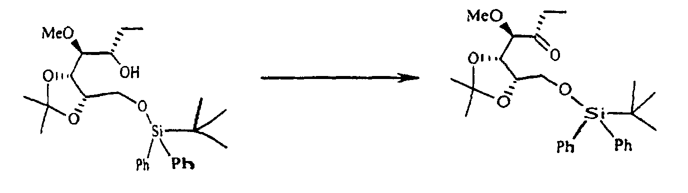

On met en suspension 22 , 57 g de pyridinium chlorochromate 0,104 moles dans 300 ml de chlorure de méthylène. On ajoute alors 110 g de tamis moléculaire 4Å. A cette suspension, on introduit 33 g de produit du stade précédent en solution dans 100 ml de chlorure de méthylène . Après 3 heures d'agitation à température ambiante, la suspension est filtrée. Le filtrat est évaporé à sec. Le résidu obtenu (35 g) est purifié sur silice avec le mélange éluant heptane 4/acétate d'éthyle 1. On obtient 27 g de produit recherché.
Rendement 83%.

### STADE D :

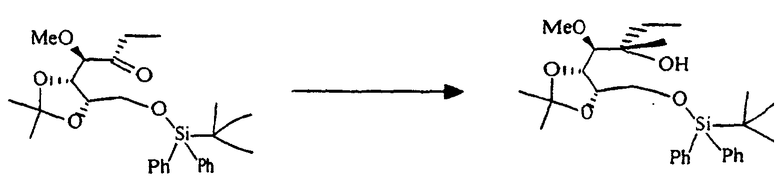

On met en solution 16,5 g de produit du stade précédent dans 150 ml de tétrahydrofurane. Sous argon, à -5°C, on ajoute goutte à goutte, 17,52 ml de solution (3M) de bromure de méthylmagnésien dans l'éther. On agite 1 heure à la température ambiante. A 0°C, on neutralise l'excès de magnésien avec une solution aqueuse de dihydrogénophosphate de sodium à 1M. On ajoute 200 ml d'un mélange d'heptane 1/acétate d'éthyle 2. La phase organique est lavée avec 200 ml d' une solution aqueuse de dihydrogénophosphate de sodium mol aire, séchée sur sulfate de magnésium et évaporée à sec . On empâte dans du pentane le produit obtenu. On obtient 14,85 g de produit recherché. Rendement : 87%

### STADE E :

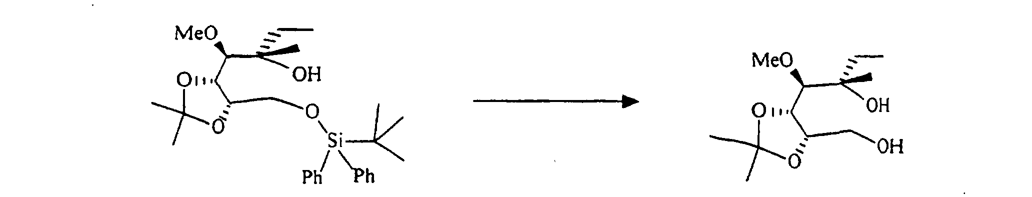

On met en solution 14,85 g de produit du stade précédent dans 150 ml de tétrahydrofurane. Sous argon, à 0°C, on ajoute goutte à goutte, 33 ml de solution molaire de fluorure de tétrabutylammonium dans le tétrahydrofurane. Après 30 minutes d'agitation à température ambiante, la réaction est terminée. On ajoute 200 ml d'un mélange d'heptane 1/acétate d'éthyle 2. La phase organique est lavée avec 200 ml d'une solution aqueuse de dihydrogénophosphate de sodium molaire, séchée sur sulfate de magnésium et évaporée à sec. Le produit brut est purifié sur silice en éluant avec le mélange chlorure de méthylène à 15% d'acétone puis à 30% d'acétone. On obtient 7,85 g de produit recherché
Rendement : Quantitatif

### STADE F :

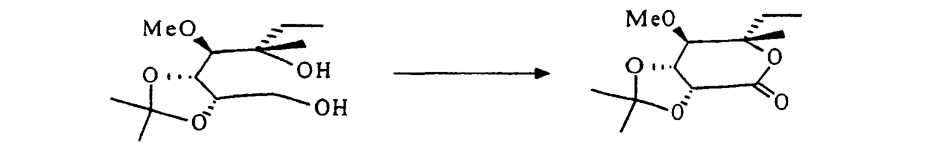

On met en solution 7,85 g de produit du stade précédent dans 82,5 ml de chlorure de méthylène. Sous argon, à température ambiante, on ajoute 44,5 ml de triéthylamine et 82,5 ml de diméthylsulfoxide stocké sur tamis moléculaire. La solution est refroidie à environ 5°C avec un bain de glace-eau et on ajoute par fraction 15,8 g de pyridine sulfur trioxide sans que la température ne dépasse 15°C. On agite pendant 1 heure. Le milieu réactionnel est versé dans 1 litre de solution aqueuse de dihydrogénophosphate de sodium molaire, la phase aqueuse est extraite avec un mélange heptane 1/acétate d'éthyle 2 .La phase organique est lavée à l'eau, séchée sur sulfate de magnésium et évaporée à sec. Le produit obtenu est empâté dans du pentane. On obtient 5,77 g de produit recherché.
Rendement 80%.

### STADE G :

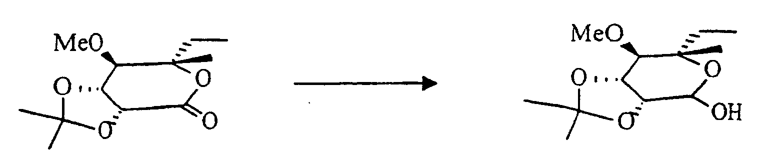

On met en solution 5,46 g de produit du stade précédent dans 25 ml de tétrahydrofurane. Sous argon, à 0°C, on ajoute 16,7 ml de solution à 1.5M de DIBAL dans le toluène.
On agite pendant 1 heure 30 à 0°C. Le milieu réactionnel est versé dans 250 ml de solution à 1 M de tartrate double de sodium et potassium ; la phase aqueuse est extraite avec un mélange heptane 1/acétate d'éthyle 2 . La phase organique est lavée avec 150 ml de solution aqueuse de sulfate de sodium à 10%, séchée sur sulfate de magnésium et évaporée à sec. On obtient 5,5 g de produit recherché. Rendement : Quantitatif

### STADE H :

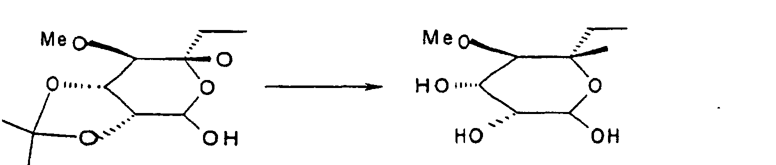

On met en émulsion 5,5 g du produit du stade précédent dans 32 ml de solution d'acide sul furique à 0, 05 N. Après une heure 30 de chauffage à 70°C, la réaction est terminée. On laisse revenir à température ambiante et le milieu réactionnel est neutralisé avec 0,6 g de carbonate de baryum ; la suspension est agitée une heure à température ambiante (pH=7), puis filtrée sur papier filtre milipore et évaporée à sec. Pour sécher le produit, on fait deux entraînements au toluène. On sèche à 40°C en présence de P₂O₅, on obtient 4,8 g de résidu gommeux blanc.

Rendement quantitatif.

### EXEMPLE 9

### Acide(2-propynyloxy)-carbamique 3'-ester de 7-[(6-déoxy-5-C-éthyl-4-O-méthyl-.bêta.-D-gulopyranosyl)oxy]-4-hydroxy-8-méthyl-3-[1- [(2-propynyloxy)imino]éthyl]-2H-1-benzopyran-2-one

### Acide (2-propynyloxy)-carbamique 3'-ester de 7-[ (6-déoxy-5-C-éthyl-4-O-méthyl-.bêta.-D-gulopyranosyl)oxy]-4-hydroxy-3-[1-(méthoxyimino]éthyl]-8-méthyl-2H-1-benzopyran-2-one

### Acide (2-propynyloxy)-carbamique 3'-ester de 7-[(6-déoxy-5-C-éthyl-4-O-méthyl-.bêta.-D-gulopyranosyl)oxy]-3-[1-(éthoxyimino)éthyl]-4-hydroxy-8-méthyl-2H-1-benzopyran-2-one

### STADE A :

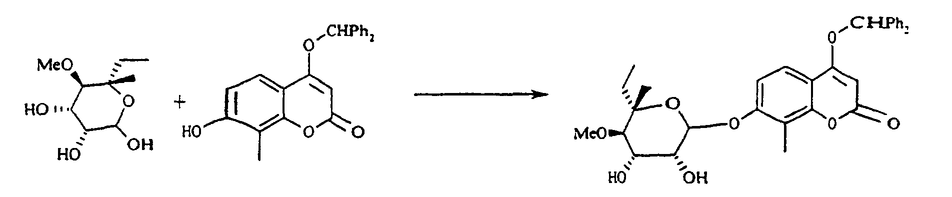

On met en solution 4,8 g du produit de la préparation 7 dans 100 ml de chlorure de méthylène . A température ambiante, sous argon, on ajoute 9,98 g de coumarine et 7,23 g de triphénylphosphine. La suspension est refroidie à 0°C, on ajoute goutte à goutte, 4,34 ml DEAD. La suspension légèrement jaune est agitée 1 heure à température ambiante . On ajoute de nouveau 6 g de triphénylphosphine et à 0°C 3, 57 ml de DEAD. On obtient une solution jaune. Après 1 heure d'agitation à température ambiante, on ajoute 50 ml de pentane pour faire précipiter le DEAD réduit. La suspension est filtrée, le filtrat est évaporé à sec et purifié sur 1, 750 Kg de silice 60 avec le mélange éluant toluène à 3% puis 6 % d'alcool isoprpylique. On obtient 10 g de cristaux blancs contenant des traces de DEAD réduit. Le produit est filtré rapidement sur silice 60 avec le mélange éluant heptane 1/acétate d'éthyle 2 pour éliminer le DEAD réduit, puis avec le mélange chlorure de méthylène à 95/méthanol 5 pour obtenir 7,3 g de produit attendu. Rendement : 58%

### STADE B :

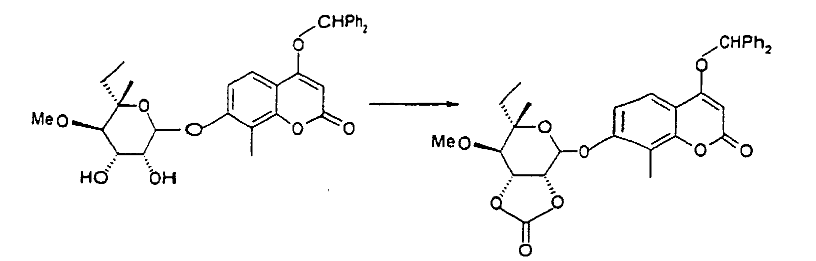

On introduit 7,2 g de produit du stade précédent dans 100 ml de THF. On ajoute 4,41 g de carbonate diimidazole et chauffe une heure au reflux. On verse dans 150 ml de solution d'hydrogénophosphate à 10% et entrait avec un mélange d'hexane acétate d'éthyle. On sèche et obtient 7,1 g de produit recherché .

### STADE C :

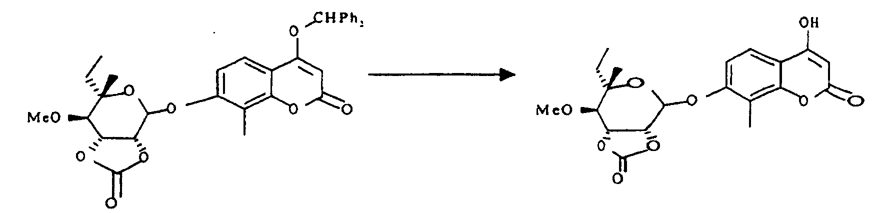

On met en solution 7,1 g de produit dans 100 ml de tétrahydrofurane. On ajoute 0,7 g de palladium sur charbon et on met sous atmosphère d'hydrogène. Après 3 heures d'agitation, la réaction est terminée. Le milieu réactionnel est filtré et le filtrat est évaporé à sec. Le produit est recristallisé dans un mélange éther/pentane. On obtient 4,75 g de produit recherché.
Rendement : 95%

### STADE D :

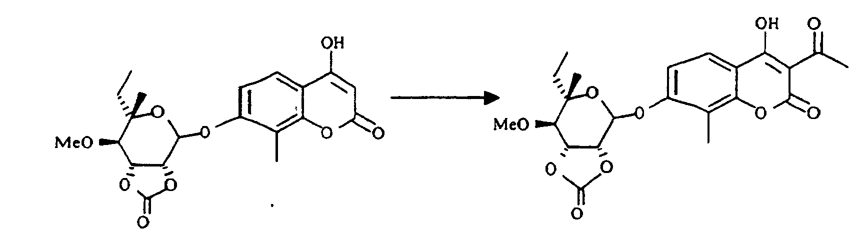

On met en solution 1,5 g de produit obtenu au stade précédent dans 25 ml de chlorure de méthylène. On ajoute 0,99 g de diméthylaminopyridine et sous argon, à 0°C, goutte à goutte, 0,38 ml d'anhydride acétique Après 30 minutes d'agitation à 0°C, on ajoute 95 µl d'anhydride acétique et 0,225 g de diméthylaminopyridine. On agite 45 minutes. Le milieu réactionnel est dilué avec 100 ml de mélange heptane 1/acétate d'éthyle 2. La phase organique est lavée avec deux fois 150 ml d'une solution aqueuse dihydrogénophosphate de sodium à 1M, séchée sur sulfate de magnésium et évaporée à sec. On isole le produit attendu. On obtient 1,56 g de produit recherché.
Rendement : 93%

### STADE E :

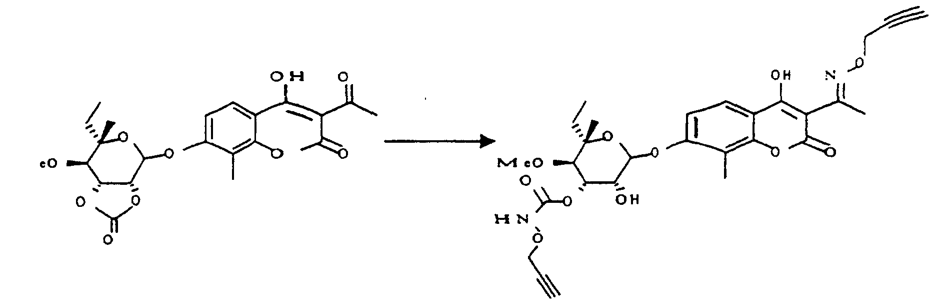

On met en solution 1,5 g de produit du stade précédent dans 15 ml de pyridine séchée sur potasse. A température ambiante, on ajoute 3,6 g du chlorhydrate de 1 a propargyl-hydroxylamine et 0,36 g de perchlorate de lithium. Le milieu réactionnel est agité 48 heures à température ambiante. On dilue avec un mélange heptane 1/acétate d'éthyle 2 et la phase organique est lavée avec une solution d'hydrogé-nosulfate de sodium à 10% séchée sur sulfate de magnésium.
On obtient 1,8 g de produit recherché. 200 mg de ce produit brut est purifié sur silice avec le mélange éluant chlorure de méthylène à 80/terbutylméthyléther 20. On obtient 90 mg de produit recherché, isomère en 3 et 75 mg d'isomère en 2
Rendement : 77% 55/45 en isomère 3

### STADE F :

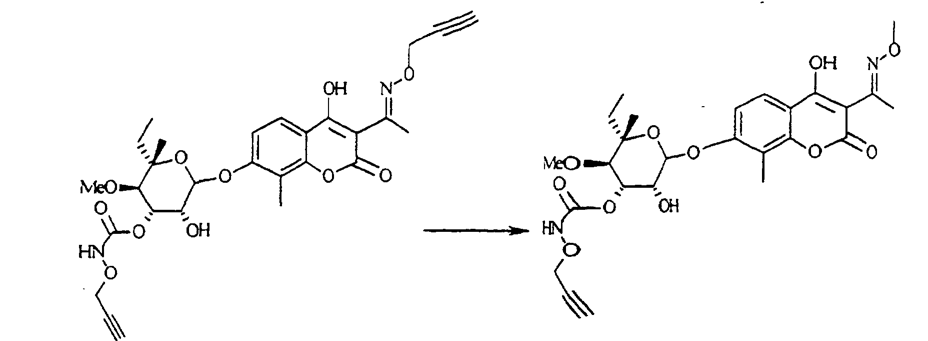

On met en solution 0,5 g de produit du stade précédent dans 5 ml d' éthanol. A température ambiante, on ajoute 0,85 g de chlorhydrate de méthylhydroxylamine et 0,94 g d'acétate de potassium. Le milieu réactionnel est agité 5 heures à température ambiante. On dilue avec un mélange heptane 1/acétate d'éthyle 2 et la phase organique est lavée avec une solution dihydrogénophophate de sodium à 1 M, séchée sur sulfate de magnésium et évaporée à sec. On obtient un produit brut qui est purifié sur silice avec le mélange éluant chlorure de méthylène à 20% de terbutylméthyléther.
On obtient : 0,095 g de produit recherché.

### STADE G :

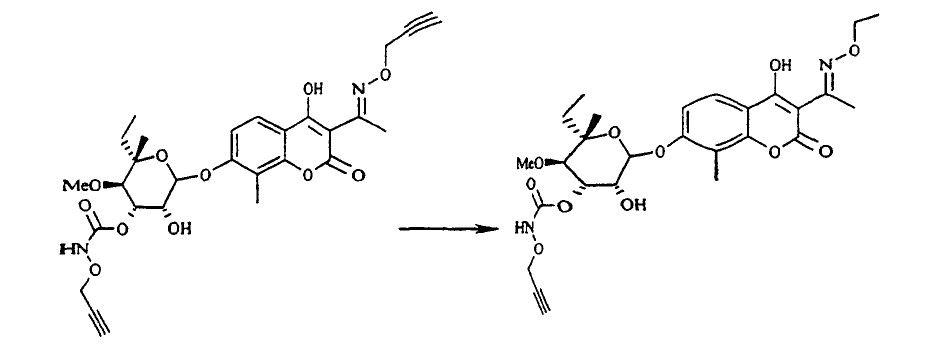

On opère comme indiqué au stade F en utilisant 0,85 g de chlorhydrate d'éthyle hydroxylamine. On obtient 0,103 g du produit attendu isomère en 3.

### EXEMPLE 10

### Acide(2-propynyloxy)-carbamique 3'-ester de 7- [(6-déoxy-5-C-éthyl-4-O-méthyl-.bêta.-D-gulopyranosyl)oxy]-3-[1-(méthoxy imino)propyl]-4-hydroxy-8-méthyl-2H-1-benzopyran-2-one

### STADE A :

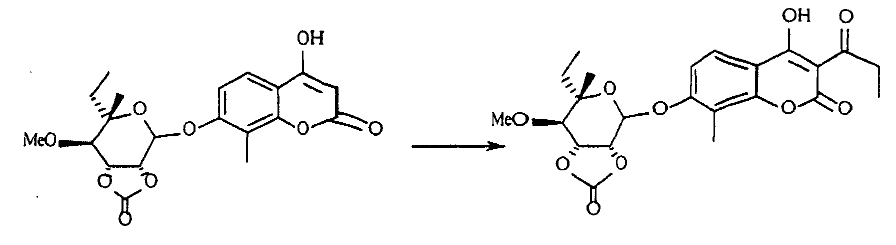

On met en solution 0, 6 g de produit obtenu comme au stade C de l'exemple 9 dans 15 ml de chlorure de méthylène. On ajoute 0,36 g de diméthylaminopyridine et sous argon, à 0°C, goutte à goutte, 0,20 g d'anhydride propionique. On agite 30 minutes à 0°C puis 1 heure à température ambiante. Le milieu réactionnel est dilué avec 100 ml de mélange heptane 1/acétate d'éthyle 2. La phase organique est lavée avec une solution aqueuse dihydrogénophosphate de sodium à 1 M, séchée sur sulfate de magnésium et évaporée à sec. On obtient 0,6 g de produit recherché.
Rendement : 68%

### STADE B :

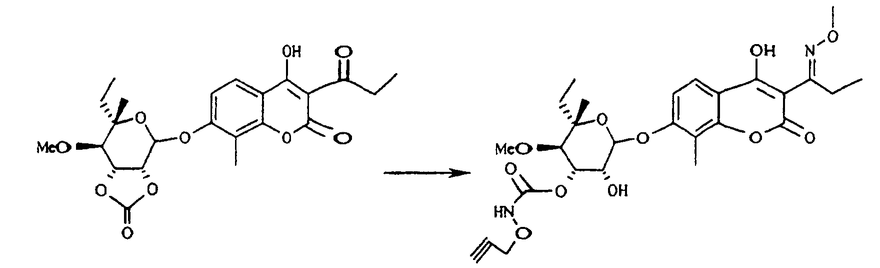

On met en solution 0,6 g de produit du stade précédent dans 6 ml de pyridine séchée sur potasse. A température ambiante, on ajoute 1,39 g du chlorhydrate de la propargylhydroxy-lamine et 0,13 g de perchlorate de lithium. Le milieu réactionnel est agité 48 heures à température ambiante. On dilue avec un mélange heptane 1/acétate d'éthyle 2 et la phase organique est lavée avec une solution d' hydrogénosulfate de sodium à 10%, séchée sur sulfate de magnésium. On obtient 0,56 g de produit que l'on met en solution dans 10 ml d'éthanol, on ajoute 1, 07 g de chlorhydrate de mé thylhydroxyl amine et 1,39 g d'acétate de sodium. Le milieu réactionnel est agité 5 heures à température ambiante. On dilue avec un mélange heptane 1/acétate d'éthyle 2 et la phase organique est lavée avec une solution dihydrogénophosphate de sodium à 1 M, séchée sur sulfate de magnésium et évaporée à sec. On obtient 0,45 g de produit brut qui est purifié par chromatographie sur silice en éluant avec le mélange chlorure de méthylène à 20% de terbutylméthyléther. On obtient 0,170 g de produit recherché. En opérant comme précédemment , on a également préparé les produits répondant à la formule

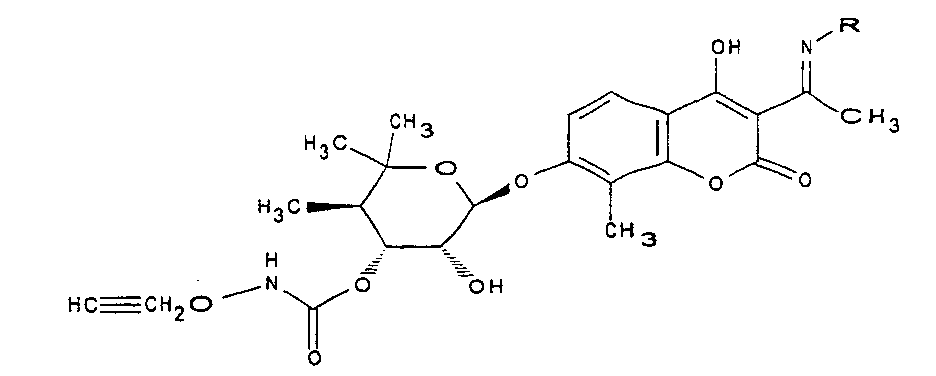

R : O(CH₂)₂Br

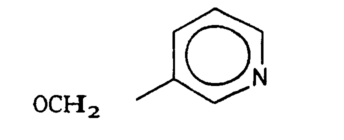

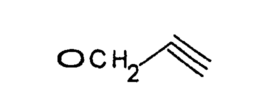

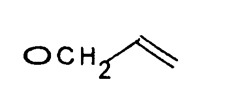

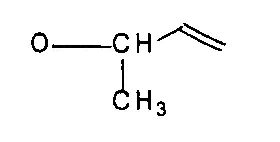

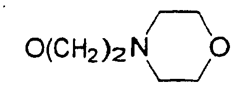

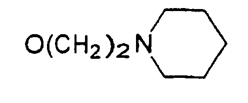

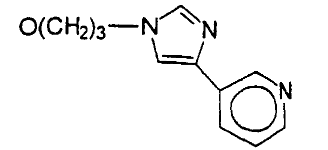

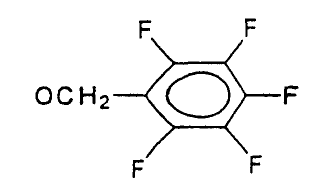

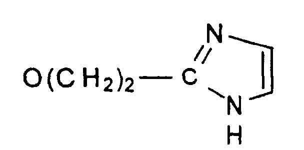

En opérant comme précédemment on a obtenu les produits suivants répondant à la formule (I)

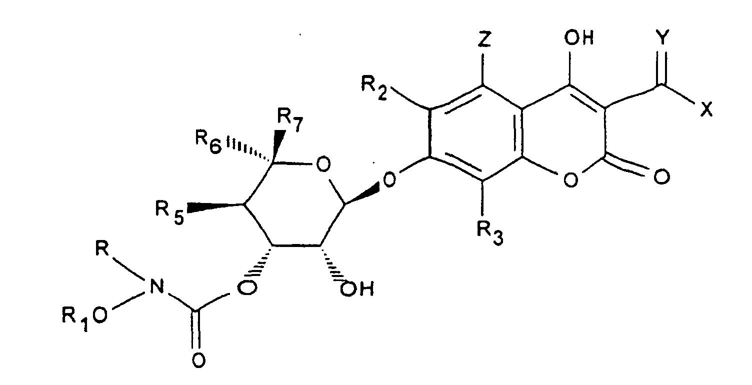

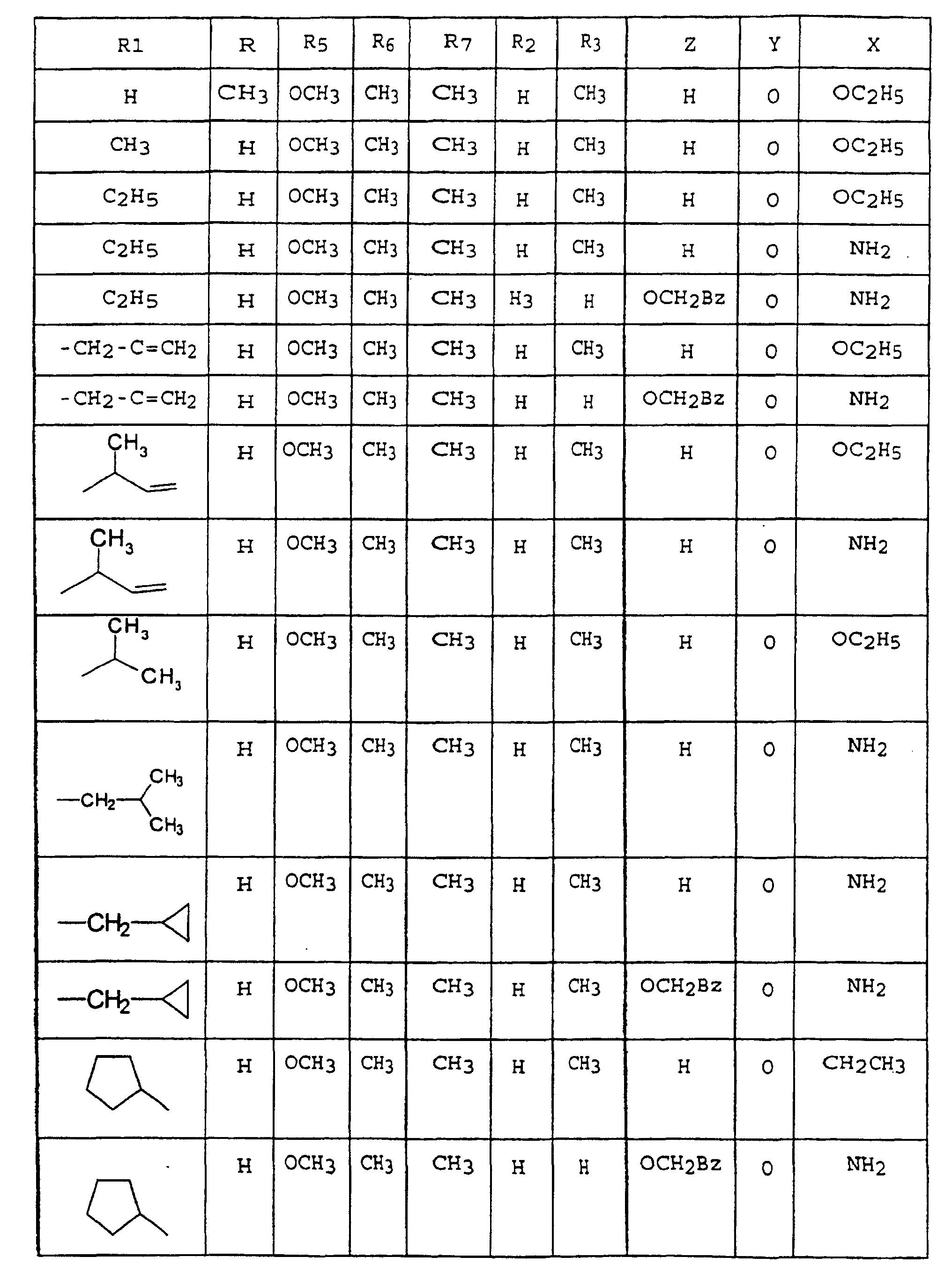

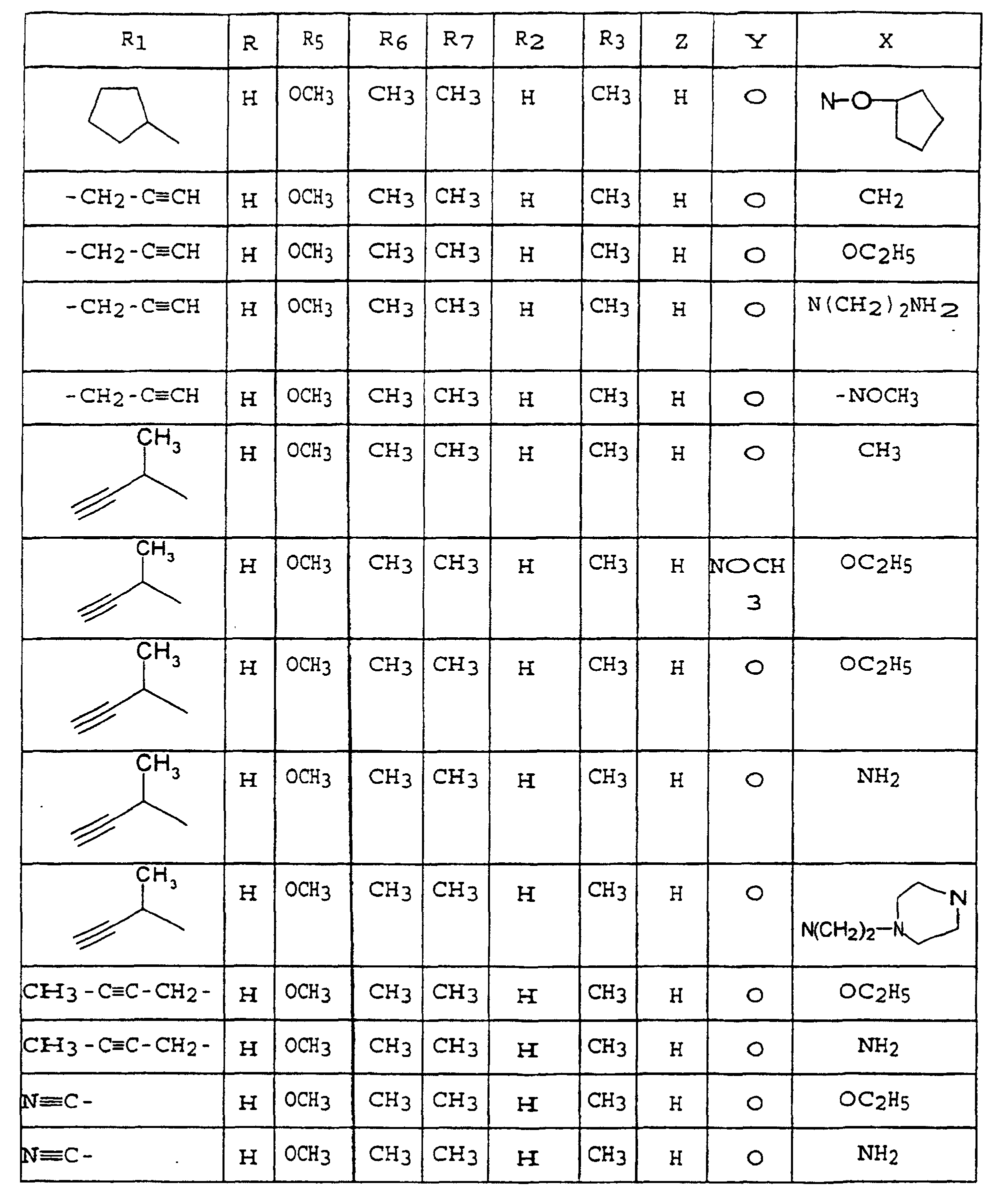

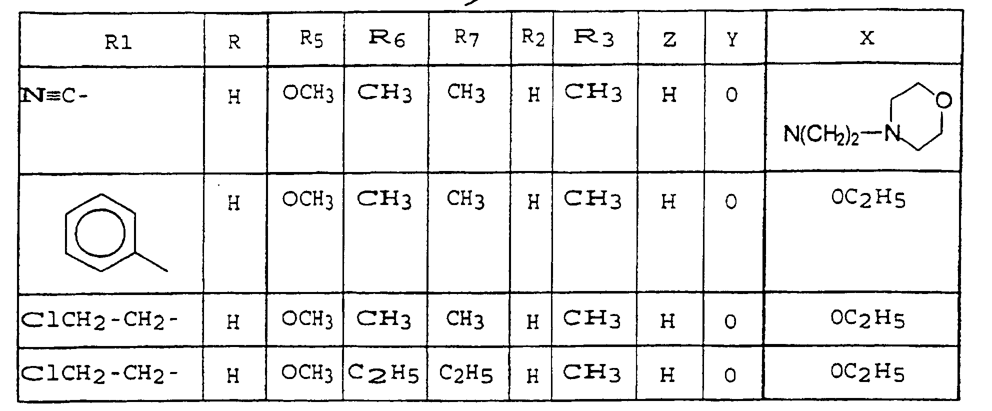

En opérant comme précédemment on a préparé les produits suivants :
**Acide(2-propynyloxy)-carbamique 3'-ester de 3-[1-[ [ (5-chloro-1,2,3-thiadiazol-4-yl)méthoxy]imino]éthyl]-7-[(6-déoxy-5-C-méthyl-4-O-méthyl-.alpha.-L-lyxo-hexopyranosyl)oxy]-4-hydroxy-8-méthyl-2H-1-benzopyran-2-one**
**Acide(2-propynyloxy)-carbamique 3'-ester de 3-[1-[(cyanométhoxy)imino]éthyl]-7- [(6-déoxy-5-C-méthyl-4-O-méthyl-.alpha.-L - lyxo-hexopyranosyl)oxy]-4-hydroxy-8-méthyl-2H-1-benzopyran-2 -one**
**Acide (2-propynyloxy)-carbamique 3'-ester de 3-[1-[(2-aminoéthoxy)imino]éthyl]-7-[(6-déoxy-5-C-méthyl-4-O-méthyl--alpha.-L-lyxo-hexopyranosyl)oxy]-4-hydroxy-8-méthyl-2H-1-benzopyran-2-one**
**Acide(2-propynyloxy)-carbamique 3'-ester de 7- [[6-déoxy-5-C-méthyl-4-O-méthyl-.alpha.-L-lyxo-hexopyranosyl)oxy]-4-hydroxy-8-méthyl-3[1-[(2-hydroxyéthoxy)imino]éthyl]-2H-1-benzopyran-2 - one**
**Acide (2-propynyloxy)-carbamique 3'-ester de 7-[(6-déoxy-5-C-méthyl-4-O-méthyl-.alpha.-L-lyxo-hexopyranosyl)oxyl]-4-hydroxy-8-méthyl-3-[1-[[(3-pipéridinyl)oxy]imino]éthyl]-2H-1-benzopyran-2-one (isomère B)**
**Acide (2-propynyloxy)-carbamique 3'-ester de 7-[(6-déoxy-5-C-** **méthyl-4-O-méthyl-.alpha.-L-lyxo-hexopyranosyl)oxy]-4-hydroxy-8-méthyl-3-[1- [[(3-pipéridinyl)oxy]imino]éthyl]-2H-1-benzopyran-2-one (isomère A)**
**Acide (2-propynyloxy)-carbamique 3'-ester de 7 - [(6-déoxy-5-C-méthyl-4-O-méthyl-.alpha.-L-lyxo-hexopyranosyl)oxy]-4-hydroxy-8-méthyl-3-[1- [ ( 1-méthylethoxy)imino]éthyl]-2H-1-benzopyran-2-one**
**Acide (2-propynyloxy)-carbamique 3'-ester de 3 - [1-[(cycobutyloxy)imino]éthyl]-7- [(6-déoxy-5-C-méthyl-4-O-méthyl-.alpha.-L-lyxo-hexopyranosyl)oxy]-4-hydroxy-8-méthyl-2H-1-benzopyran-2-one**
**Acide (2-propynyloxy)-carbamique 3'-ester de 7-[(6-déoxy-5-C-méthyl-4-O-méthyl-.alpha.-L-lyxo-hexopyranosyl)oxy]-4-hydroxy-8-méthyl-3-[1-(propoxyimino)éthyl]-2H-1-benzopyran-2-one**
**Acide (2-propynyloxy)-carbamique 3'-ester de 7-[(6-déoxy-5-C-méthyl-4-O-méthyl-.alpha.-L-lyxo-hexopyranosyl)oxy]-4-hydroxy-8-méthyl-3-[1-[2,2,2-trifluoroéthoxy)imino]éthyl]-2H-1-benzopyran-2-one**
**Acide (2-propynyloxy)-carbamique 3'-ester de 7-[(6-déoxy-5-C-méthyl-4-O-méthyl-.alpha.-L-lyxo-hexopyranosyl)oxy]-4-hydroxy-8-méthyl-3-[1-[[(pentafluorophényl)méthoxy]imino] éthyl]-2H-1-benzopyran-2-one**
**Acide (2-propynyloxy)-carbamique 3'-ester de 7 - [(6-déoxy-5-C-méthyl-4-O-méthyl-.alpha.-L-lyxo-hexopyranosyl)oxy]-4-hydroxy-8-méthyl-3-[1- [ [3-[4- (3-pyridinyl)-1H-imidazol-1-yl] propoxy]imino]éthyl] - 2H-1-benzopyran-2-one**
**Acide (2-propynyloxy)-carbamique 3'-ester de 7-[(6-déoxy-5-C-méthyl-4-O-méthyl-alpha.-L-lyxo-hexopyranosyl)oxy]-4-hydroxy-8-méthyl-3-[1- [[2-(1-piperidinyl)éthoxy]-imino] éthyl]-2H-1-benzopyran-2-one**
**Acide (2-propynyloxy)-carbamique 3'-ester de 7-[(6-déoxy-5-C-méthyl-4-O-méthyl-.alpha.-L-lyxo-hexopyranosyl)oxy]-4-** **hydroxy-8-méthyl-3-[1-[[2-(4-morpholinyl)éthoxy]-imino]éthyl]-2H-1-benzopyran-2-one**
**Acide(2-propynyloxy)-carbamique 3'-ester de 7-[(6-déoxy-5-C-méthyl-4-O-méthyl-.alpha.-L-lyxo-hexopyranosyl)oxy] - 4 - hydroxy-8-méthyl-3-(1-(méthoxyimino)propyl]-2H-1-benzopyran-2-one**
**Acide (2-propynyloxy) - carbamique 3' - ester de 7-[(6-déoxy-5-C-méthyl-4-O-méthyl-.alpha.-L-lyxo-hexopyranosyl)oxy]-4-hydroxy-8-méthyl-3-[1-[(2,2,2-trifluoroéthoxy)imino)propyl]-2H- 1-benzopyran-2-one**
**Acide(2-propynyloxy)-carbamique 3'-ester de 7-[(6-déoxy-5-C-méthyl-4-O-méthyl-.alpha.-L-lyxo-hexopyranosyl)oxy] - 4-hydroxy-8-méthyl-3-[1- (prppoxyimino) propyl]-2H-1-benzopyran-2-one**
**Acide(2-propynyloxy)-carbamique 3'-ester de 7-[(6-déoxy-5-C-méthyl-4-O-méthyl-.alpha.-L-lyxo-hexopyranosyl)oxy]-3-[1-(éthoxyimino)propyl]-4-hydroxy-8-méthyl-2H-1-benzopyran-2-one 7-[[6-déoxy-5-C-méthyl-4-O-méthyl-3-O-[[(2-propynyloxy) amino]carbonyl] - . alpha.-L-lyxo-hexopyranosyl)oxy]-3-[1-(éthoxyméthoxy)imino]éthyl]-4-hydroxy-8-méthyl-2H-1-benzopyran-2 - one**
**7- [[6-déoxy-5-C-méthyl-4-O-méthyl-3-O-[[(2-propynyloxy) propynyloxy)amino]carbonyl].alpha.-L-lyxo-hexopyranosyl) oxy]-4-hydroxy-8-méthyl-3- [1-[ [ (2-méthyl-4-thiazolyl)méthoxy] imino]éthyl]-2H-1-benzopyran-2-one**
**Acide (2-propynyloxy)-carbamique 3'-ester de 7-[(6-déoxy-5-C-méthyl-4-O-méthyl-.alpha.-L-lyxo-hexopyranosyl)oxy]-4-hydroxy-8-méthyl-3-[1-[[(2-thiazolyl)méthoxy]imino]éthyl]-2H-1-benzopyran-2-one**
**Acide (2-propynyloxy)-carbamique 3'-ester de 7- [(6-déoxy-5-C-méthyl-4-O-méthyl-.alpha.-L-lyxo-hexopyranosyl)oxy]-4-hydroxy-8-méthyl-3-[1-[[(3-furanyl)méthoxy]imino]éthyl]-2H-1-benzopyran-2 - one**
**Acide(2-propynyloxy)-carbamique 3'-ester de 7-[(6-déoxy-5-C-méthyl-4-O-méthyl-.alpha.-L-lyxo-hexopyranosyl)oxy]-4-hydroxy-8-méthyl-3-[1- [[(3-thiényl)méthoxy]imino]éthyl]-2H-1-benzopyran-2-one**
**Acide(2-propynyloxy)-carbamique 3'-ester de 7- [(6-déoxy-5-C-méthyl-4-O-méthyl-.alpha.-L-lyxo-hexopyranosyl)oxy]-4-hydroxy-3-[1-[[(2-furanylméthoxy)imino]éthyl]-8-méthyl-2H-1-benzopyran-2-one**
**Acide(2-propynyloxy)-carbamique 3'-ester de 7-[(6-déoxy-5-C-méthyl-4-O-méthyl-.alpha.-L-lyxo-hexopyranosyl)oxy]-4-hydroxy-3-[1-[[(3,5-diméthyl-isoxazol-4-yl)méthoxy]imino] éthyl]-8-méthyl-2H-1-benzopyran-2-one**
**Acide(2-propynyloxy)-carbamique 3'-ester de 7- [(6-déoxy-5-C-méthyl-4-O-méthyl-..alpha.-L-lyxo-hexopyranosyl)oxy]-4-hydroxy-8-méthyl-3-[1-(phénoxyimino)éthyl]-2H-1-benzopyran-2-one**
**[[[1-[7-[[6-déoxy-5-C-méthyl-4-O-méthyl-3-O-[[(2-propynyloxy)amino]carbonyl]-.alpha.-L-lyxo-hexopyranosyl) oxy]-4-hydroxy-8-méthyl-2-oxo-2H-1-benzopyran-3-yl]éthylidene]amino]oxy] acétate de méthyle**

### EXEMPLES DE COMPOSITIONS PHARMACEUTIQUES

On a préparé des comprimés renfermant :

| | |
|---|---|
| **Produit de l'exemple 1** | 150 mg |
| Excipient q.s.p. | 1 g |
| Détail de l'excipient : amidon, talc, stéarate de magnésium | |

| | |
|---|---|
| **Produit de l'exemple 5** | 150 mg |
| Excipient q.s.p | 1 g |
| Détail de l'excipient : amidon, talc, stéarate de magnésium | |

On a également préparé des solutions injectables à partir des produits salifiés.

### ETUDE PHARMACOLOGIQUE DES PRODUITS DE L'INVENTION

### A - Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37 C, l'inhibition de la croissance est appréciée par transillumination de ce qui permet de déterminer les concentrations minimales inhibitrices (C.M.I.) exprimées en microgrammes/cm3.
Activité in vitro
CM1 en µg/ml
Sur les souches suivantes :

| | Ex.1 | Ex.2 | Ex.3 | Ex.4 |
|---|---|---|---|---|
| Staph. aureus 011HT18 | 0,04 | 0,04 | 0,04 | 0,04 |
| Staph. epidermidis 0126042 | 0,04 | 0,04 | 0,04 | 0,15 |
| Staph. coag. négative 012HT5 | 0,3 | 0,04 | 0,15 | 0,15 |
| Strepto. pyogene 02A1UC1 | 0,6 | 0,3 | 0,6 | 0,6 |
| Strepto. pneumoniae 030BI2 | 0,04 | 0,08 | 0,08 | 0,15 |
| Entero faecium 02D3IP2 | 0,08 | 0,6 | 1,2 | 1,2 |
| Entero faecalis 02D2UC5 | 0,3 | 1,2 | 1,2 | 1,2 |

### B - Inhibition de la gyrase B

Les produits sont des inhibiteurs de gyrase B ; la dose à 50% du surenroulement de l'ADN est inférieure à 5 µg/ml.

## Revendications

1. Les composés de formule (I) : 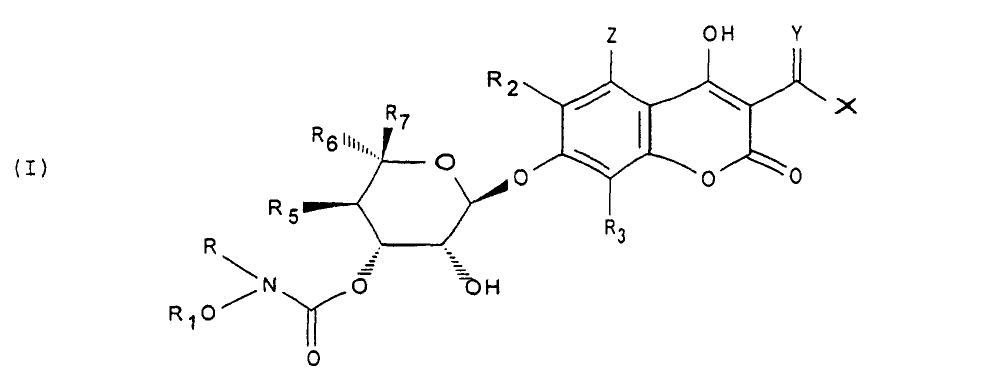 dans laquelle :
- Y représente un atome d'oxygène, ou un radical N-Nalc₁ ou NOalc₂ dans lequel alc₁ et alc₂ représentent un radical alkyle, renfermant jusqu'à 12 atomes de carbone éventuellement interrompu par un ou plusieurs atomes d'oxygène, de soufre ou d'azote éventuellement substitué par un ou plusieurs atomes d'halogène, par un radical aryle éventuellement substitué par un ou plusieurs atomes d'halogène, par un radical hétérocyclique, par un ou plusieurs radicaux 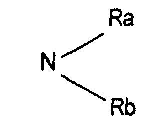
dans lequel Ra et Rb identiques ou différents l'un de l'autre représentent un atome d'hydrogène, un radical alkyle renfermant jusqu'à 8 atomes de carbone éventuellement substitué, ou Ra et Rb forment ensemble avec l'atome d'azote auxquels ils sont joints un hétérocycle pouvant renfermer en outre un autre hétéroatome choisi parmi
l'oxygène, le soufre ou l'azote,
X représente un atome d'hydrogène, un radical hydroxyle, un radical alkyle, alkényle ou alkynyle éventuellement interrompu par un ou plusieurs atomes d'oxygène, de soufre et
ou d'azote, linéaire, ramifié ou cyclique, renfermant jusqu'à 12 atomes de carbone, éventuellement substitué par un ou plusieurs atomes d'halogène, par un radical hétérocyclique, un ou plusieurs radicaux OH libres ou estérifiés, C≡N, NO₂, 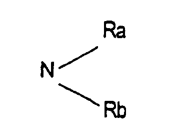 dans lequel Ra et Rb, identiques ou différents, représentent un atome d'hydrogène, un radical alkyle renfermant jusqu'à 8 atomes de carbone, ou Ra et Rb forment avec l'atome d'azote auquel ils sont Liés un hétérocycle renfermant éventuellement un autre hétéroatome choisi parmi l'azote, le soufre ou l'oxygène, ou X représente un radical alkoxy ou un radical 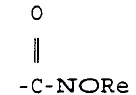 dans lequel Re représente un radical alkyle renfermant jusqu'à 8 atomes de carbone, éventuellement substitué par un ou plusieurs des substituants indiqués ci-dessus,
ou X représente un radical NRcRd dans lequel Rc et Rd identiques ou différents, représentent un atome d' hydrogène ou un radical alkyle renfermant jusqu'à 12 atomes de carbone, éventuellement substitué par un ou plusieurs des substituants indiqués ci-dessus, ou Rc et Rd forment ensemble avec l'atome d'azote auquel ils sont liés un hétérocycle renfermant éventuellement un autre hétéroatome choisi parmi l'azote, le soufre ou l'oxygène,
- Z représente un atome d'hydrogène ou d'halogène ou un radical OH libre, éthérifié ou estérifié,
- R₂ représente un atome d'hydrogène ou d'halogène,
- R₃ représente un atome d'hydrogène, un radical alkyle renfermant jusqu'à 8 atomes de carbone ou un atome
- d'halogène,
- R représente un atome d'hydrogène ou un radical alkyle renfermant jusqu'à 4 atomes de carbone,
- R₁ représente un atome d'hydrogène, un radical alkyle, alkényle ou alkynyle linéaire, ramifié ou cyclique renfermant jusqu'à 8 atomes de carbone, éventuellement substitué par un
ou plusieurs atomes d'halogène, un radical C≡N, un radical aryle renfermant jusqu'à 14 atomes de carbone,
- R₅ représente un atome d'hydrogène, un radical O-alkyle renfermant jusqu'à 4 atomes de carbone,
- ou bien R₆ représente un radical alkyle ou CH₂-O-alkyle, dans lequel alkyle représente un radical alkyle renfermant jusqu'à 8 atomes de carbone, éthényle ou 2-propényle,
- R₇ représente un atome d'hydrogène ou un radical alkyle renfermant jusqu'à 8 atomes de carbone,
- ou bien R₆ et R₇ forment avec l'atome de carbone qui les portent, un cycle renfermant jusqu'à 8 atomes de carbone, ainsi que les sels du composé de formule (I), lorsque les composés de formule (I) ont une fonction basique.

2. Les composés de formule (I) définis à la revendication 1, dans lesquels Y représente un atome d'oxygène.

3. Les composés de formule (I) dans lesquels Y représente un radical NO-alkyle dans lequel le radical alkyle renferme jusqu'à 4 atomes de carbone.

4. Les composés de formule (I) définis à la revendication 3, dans lesquels Y représente le radical NOC₂H₅.

5. Les composés de formule (I) définis à l'une quelconque des revendications 1 à 4 dans lesquels X représente un radical alkyle renfermant jusqu'à 4 atomes de carbone et notamment le radical CH₃.

6. Les composés de formule (I) définis à l'une quelconque des revendications 1 à 4, dans lesquels X représente un radical NH₂.

7. Les composés de formule (I) définis à l'une quelconque des revendications 1 à 4 dans lesquels X représente le radical :

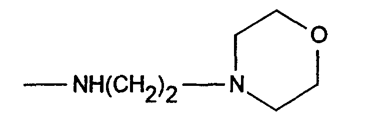

8. Les composés de formule (I) définis à l'une quelconque des revendications 1 à 7 dans lesquels R₁ représente un radical :
HC≡C-CH₂-

9. Les composés de formule (I) définis à l'une quelconque des revendications 1 à 8 dans lesquels R représente un atome d'hydrogène.

10. Les composés de formule (I) définis à l'une quelconque des revendications 1 à 9 dans lesquels R₃ représente un radical méthyle .

11. Les composés de formule (I) définis à l'une quelconque des revendications 1 à 10 dans lesquels Z représente un atome d'hydrogène.

12. Les composés de formule (I) définis à l'une quelconque des revendications 1 à 11 dans lesquels R₂ représente un atome d'hydrogène.

13. Les composés de formule (I) définis à l'une quelconque des revendications 1 à 12 dans lesquels R₅ représente un radical OCH₃.

14. Les composés de formule (I) définis à l'une quelconque des revendications 1 à 13 dans lesquels R₆ représente un radical méthyle.

15. Les composés de formule (I) définis à l'une quelconque des revendications 1 à 14 dans lesquels R₇ représente un radical méthyle.

16. Les composés de formule (I) définis à l'une quelconque des revendications 1 à 14 dans lesquels R₇ représente un radical éthyle.

17. Les composés de formule (I), définis à l'une quelconque des revendications 1 à 13 dans lesquels R₆ et R₇ forment avec l'atome de carbone qui les porte un radical cyclopentyle.

18. Les composés de formule (I) définis à la revendication 1 dont les noms suivent :
- acide (2-propynyloxy)carbamique 3' ester de 7- [[6-déoxy-5-C-méthyl-4-O-méthyl-alpha-L-lyxo-hexopyranosyl]oxy] -4-hydroxy-8-méthyl-2-oxo-2H-1-benzopyran-3-carboxamide
- acide (2-propynyloxy)-carbamique 3'-ester de 7-[(6-déoxy-5-C-méthyl-4-O-méthyl-alpha-L-lyxo-hexopyranosyl)oxy] - 4-hydroxy-8-méthyl-N-[2-(4-morpholinyl) éthyl]-2-oxo-2H-1-benzopyran-3-carboxamide
- acide (2-propynyloxy)-carbamique 3' -ester de 7-[[6-déoxy-5-C-méthyl-4-O-méthyl-alpha-L-lyxo-hexopyranosyl]oxy]-4 - hydroxy-3-[1-(méthoxyimino)éthyl]-8-méthyl-2H-1-benzopyran-2-one
- acide (2-propynyloxy)-carbamique 3' -ester de 7-[(6-déoxy-5-C-méthyl-4-O-méthyl-alpha-L-lyxo-hexopyranosyl)oxy]-3-[1-(éthoxyimino)éthyl]-4-hydroxy-8-méthyl-2H-1-benzopyran-2-one.

19. Les composés de formule (I) dont les noms suivent :
- Acide(2-propynyloxy)-carbamique 3'-ester de 7-[(6-déoxy-5-C-éthyl-4-O-méthyl-.bêta.-D-gulopyranosyl)oxy]-4-hydroxy-3-[1-(méthoxyimino)éthyl]-8-méthyl-2H-1-benzopyran-2-one
- [7R-(7.alpha.,8.bêta.,9.bêta.,10.alpha.)]-(2-propynyloxy)-carbamate de 8-hydroxy-7- [4-hydroxy-3 - [1-(méthoxyimino) éthyl]-8-méthyl-2-oxo-2H-1-benzopyran-7-yl]-10-méthoxy-6-oxaspiro[4.5]décan-9-yle.

20. A titre de médicaments, les composés de formule (I) définis à l'une quelconque des revendications 1 à 18 ainsi que leurs sels pharmaceutiquement acceptables.

21. A titre de médicaments les composés de formule (I) définis à la revendication 19 ainsi que leurs sels pharmaceutiquement acceptables.

22. Les compositions pharmaceutiques renfermant comme principe actif au moins un médicament défini à la revendication 20 ou 21

23. Procédé de préparation des composés de formule (I) définis à l'une quelconque des revendications 1 à 19 **caractérisé en ce que** l'on soumet un composé de formule (II) 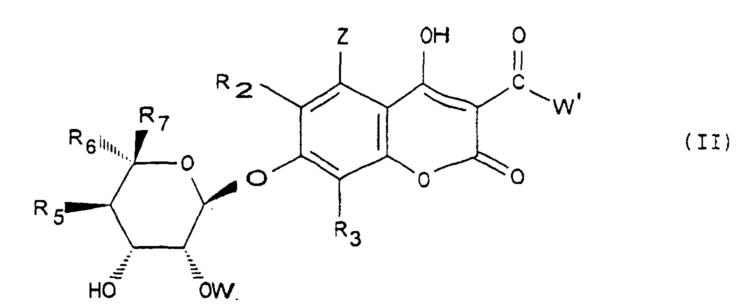 dans laquelle les radicaux R₂, R₃, Z, R₅, R₆ et R₇ conservent leur signification précédente, OW représente un groupement hydroxyle bloqué et W' représente un radical alkyle ou Oalkyle renfermant jusqu'à 4 atomes de carbone,
- à l'action un agent susceptible d'introduire le radical 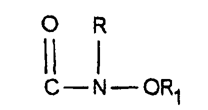
ou d'une suite d'opérations susceptibles d'introduire le radical 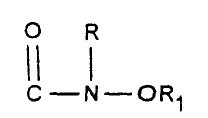 R et R₁ conservant leur signification précédente,
- à l'action d'un agent susceptible de libérer le radical hydroxyle du radical OW,
- à l'action éventuelle d'un agent susceptible de remplacer W' par le radical X différent de alkyle ou Oalkyle,
- à l'action éventuelle d' un agent susceptible d'introduire le radical Y différent de l'oxygène,
- à l'action éventuelle d' un agent de salification.

24. A titre de produits chimiques nouveaux les composés de formule (II) définis à la revendication 23.

25. Procédé selon la revendication 23 **caractérisé en ce que** le produit de formule (II) est préparé par action d'un composé de formule (III) 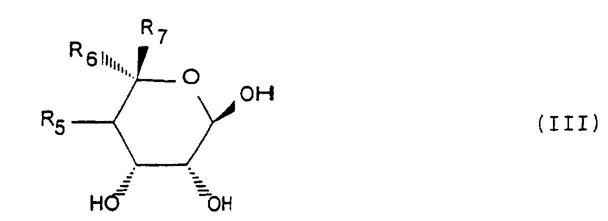 dans lequel R₅, R₆ et R₇ conservent leur signification précédente sur un composé de formule (IV) 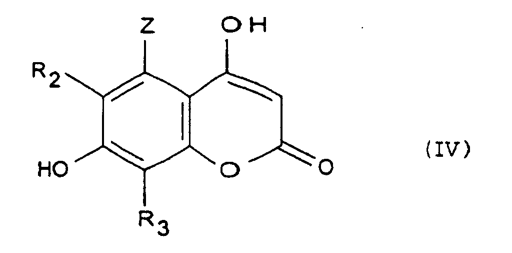 dans lequel R₂, R₃ et Z conservent leur signification précédente, puis d'un agent de blocage du radical hydroxyle libre.

26. A titre de produits chimiques nouveaux, les composés de formule (III) définis à la revendication 25 suivants

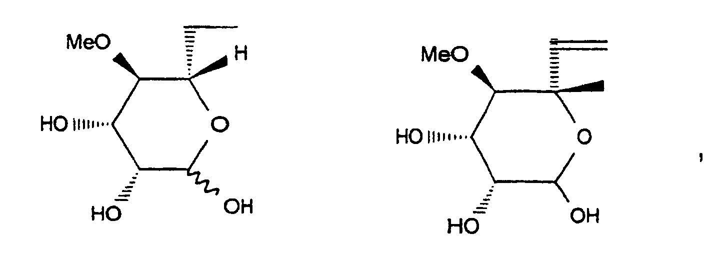

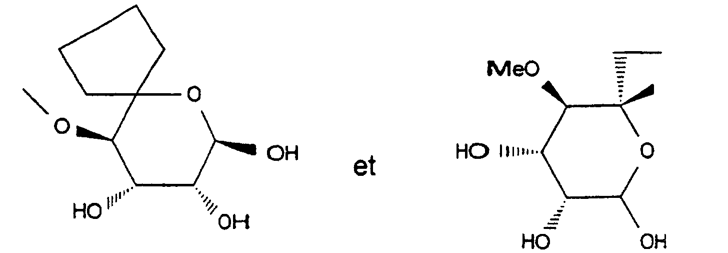

## Claims

1. The compounds of formula (I): 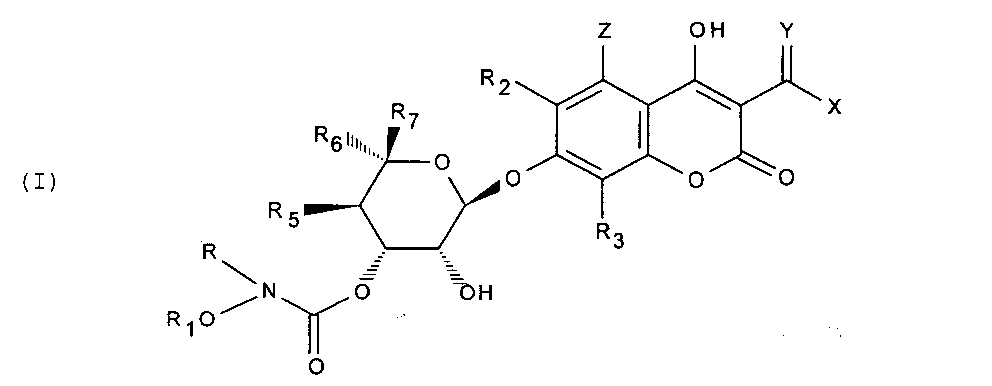 in which:
- Y represents an oxygen atom, or an N-Nalk₁ or NOalk₂ radical in which alk₁ and alk₂ represent an alkyl radical, containing up to to 12 carbon atoms optionally interrupted by one or more oxygen, sulphur or nitrogen atoms, optionally substituted by one or more halogen atoms, by an aryl radical optionally substituted by one or more halogen atoms, by a heterocyclic radical, by one or more 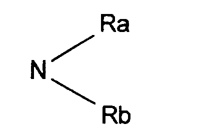 radicals
in which Ra and Rb identical to or different from one another represent a hydrogen atom, an optionally substituted alkyl radical containing up to 8 carbon atoms, or Ra and Rb form together with the nitrogen atom to which they are joined a heterocycle which can contain in addition another heteroatom chosen from
oxygen, sulphur or nitrogen,
X represents a hydrogen atom, a hydroxyl radical, a linear, branched or cyclic alkyl, alkenyl or alkynyl radical optionally interrupted by one or more oxygen, sulphur and or nitrogen atoms, containing up to 12 carbon atoms, optionally substituted by one or more halogen atoms, by a heterocyclic radical, one or more free or esterified OH, C≡N, NO₂, 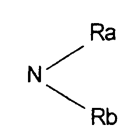 radicals in which Ra and Rb, identical or different, represent a hydrogen atom, an alkyl radical containing up to 8 carbon atoms, or Ra and Rb form together with the nitrogen atom to which they are linked a heterocycle optionally containing another heteroatom chosen from nitrogen, sulphur or oxygen, or X represents an alkoxy radical or a 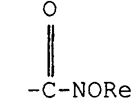 radical in which Re represents an alkyl radical containing up to 8 carbon atoms, optionally substituted by one or more of the substituents indicated above,
or X represents an NRcRd radical in which Rc and Rd identical or different, represent a hydrogen atom or an alkyl radical containing up to 12 carbon atoms, optionally substituted by one or more of the substituents indicated above, or Rc and Rd form together with the nitrogen atom to which they are linked a heterocycle optionally containing another heteroatom chosen from nitrogen, sulphur or oxygen,
- Z represents a hydrogen or halogen atom or a free, etherified or esterified OH radical,
- R₂ represents a hydrogen or halogen atom,
- R₃ represents a hydrogen atom, an alkyl radical containing up to 8 carbon atoms or a
- halogen atom,
- R represents a hydrogen atom or an alkyl radical containing up to 4 carbon atoms,
- R₁ represents a hydrogen atom, a linear, branched or cyclic alkyl, alkenyl or alkynyl radical containing up to 8 carbon atoms, optionally substituted by one or more halogen atoms, a C≡N radical, an aryl radical containing up to 14 carbon atoms,
- R₅ represents a hydrogen atom, an O-alkyl radical containing up to 4 carbon atoms,
- either R₆ represents an alkyl or CH₂-O-alkyl radical, in which alkyl represents an alkyl radical containing up to 8 carbon atoms, ethenyl or 2-propenyl,
- R₇ represents a hydrogen atom or an alkyl radical containing up to 8 carbon atoms,
- or R₆ and R₇ form together with the carbon atom which carries them, a ring containing up to 8 carbon atoms, as well as the salts of the compound of formula (I), when the compounds of formula (I) have a basic function.

2. The compounds of formula (I) defined in claim 1, in which Y represents an oxygen atom.

3. The compounds of formula (I) in which Y represents an NO-alkyl radical in which the alkyl radical contains up to 4 carbon atoms.

4. The compounds of formula (I) defined in claim 3, in which Y represents the NOC₂H₅ radical.

5. The compounds of formula (I) defined in any one of claims 1 to 4 in which X represents an alkyl radical containing up to 4 carbon atoms and in particular the CH₃ radical.

6. The compounds of formula (I) defined in any one of claims 1 to 4, in which X represents an NH₂ radical.

7. The compounds of formula (I) defined in any one of claims 1 to 4 in which X represents the: 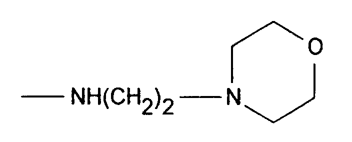 radical.

8. The compounds of formula (I) defined in any one of claims 1 to 7 in which R₁ represents a:
HC≡C-CH₂-
radical.

9. The compounds of formula (I) defined in any one of claims 1 to 8 in which R represents a hydrogen atom.

10. The compounds of formula (I) defined in any one of claims 1 to 9 in which R₃ represents a methyl radical.

11. The compounds of formula (I) defined in any one of claims 1 to 10 in which Z represents a hydrogen atom.

12. The compounds of formula (I) defined in any one of claims 1 to 11 in which R₂ represents a hydrogen atom.

13. The compounds of formula (I) defined in any one of claims 1 to 12 in which R₅ represents an OCH₃ radical.

14. The compounds of formula (I) defined any one of claims 1 to 13 in which R₆ represents a methyl radical.

15. The compounds of formula (I) defined in any one of claims 1 to 14 in which R₇ represents a methyl radical.

16. The compounds of formula (I) defined in any one of claims 1 to 14 in which R₇ represents an ethyl radical.

17. The compounds of formula (I), defined in any one of claims 1 to 13 in which R₆ and R₇ form with the carbon atom which carries them a cyclopentyl radical.

18. The compounds of formula (I) defined in claim 1 the names of which follow:
- (2-propynyloxy)carbamic acid 3'-ester of 7-[[6-deoxy-5-C-methyl-4-O-methyl-alpha-L-lyxo-hexopyranosyl]oxy]-4-hydroxy-8-methyl-2-oxo-2H-1-benzopyran-3-carboxamide
- (2-propynyloxy)-carbamic acid 3'-ester of 7-[(6-deoxy-5-C-methyl-4-O-methyl-alpha-L-lyxo-hexopyranosyl)oxy]-4-hydroxy-8-methyl-N-[2-(4-morpholinyl)ethyl]-2-oxo-2H-1-benzopyran-3-carboxamide
- (2-propynyloxy)-carbamic acid 3'-ester of 7-[[6-deoxy-5-C-methyl-4-O-methyl-alpha-L-lyxo-hexopyranosyl]oxy]-4-hydroxy-3-[1-(methoxyimino)ethyl]-8-methyl-2H-1-benzopyran-2-one
- (2-propynyloxy)-carbamic acid 3'-ester of 7-[(6-deoxy-5-C-methyl-4-O-methyl-alpha-L-lyxo-hexopyranosyl)oxy]-3-[1-(ethoxyimino)ethyl]-4-hydroxy-8-methyl-2H-1-benzopyran-2-one.

19. The compounds of formula (I) the names of which follow:
- (2-propynyloxy)-carbamic acid 3'-ester of 7-[(6-deoxy-5-C-ethyl-4-O-methyl-.beta.-D-gulopyranosyl)oxy]-4-hydroxy-3-[1-(methoxyimino)ethyl]-8-methyl-2H-1-benzopyran-2-one
- [7R-(7.alpha.,8.beta.,9.beta.,10.alpha.)]-(2-propynyloxy)-carbamate of 8-hydroxy-7-[4-hydroxy-3-[1-(methoxyimino) ethyl]-8-methyl-2-oxo-2H-1-benzopyran-7-yl]-10-methoxy-6-oxaspiro[4.5]decan-9-yl.

20. As medicaments, the compounds of formula (I) defined in any one of claims 1 to 18 as well as their pharmaceutically acceptable salts.

21. As medicaments the compounds of formula (I) defined in claim 19 as well as their pharmaceutically acceptable salts.

22. The pharmaceutical compositions contain at least one medicament defined in claims 20 or 21 as active ingredient.

23. Process for the preparation of the compounds of formula
(I) defined in any one of claims 1 to 19 **characterized in that** a compound of formula (II) 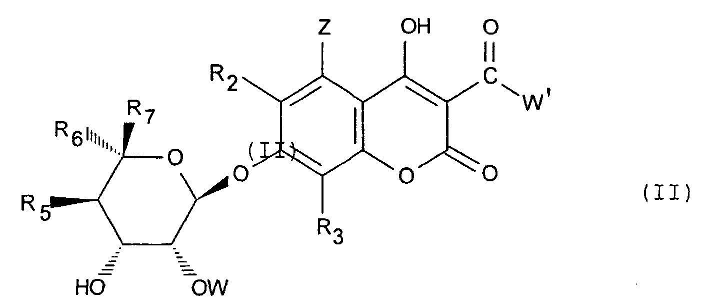 in which the R₂, R₃, Z, R₅, R₆ and R₇ radicals retain their previous meaning, OW represents a blocked hydroxyl group and W' represents an alkyl or Oalkyl radical containing up to 4 carbon atoms, is subjected
- to the action of an agent capable of introducing the 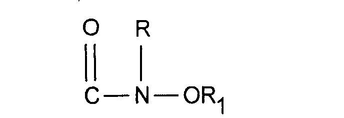 radical
or of a series of operations capable of introducing the 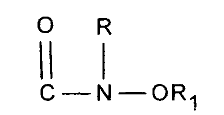 radical
R and R₁ retaining their previous meaning,
- to the action of an agent capable of releasing the hydroxyl radical from the OW radical,
- to the optional action of an agent capable of replacing W' by the X radical which is different from alkyl or Oalkyl,
- to the optional action of an agent capable of introducing the Y radical which is different from oxygen,
- to the optional action of a salification agent.

24. As new chemical products the compounds of formula (II) defined in claim 23.

25. Process according to claim 23 **characterized in that** the product of formula (II) is prepared by the action of a compound of formula (III) 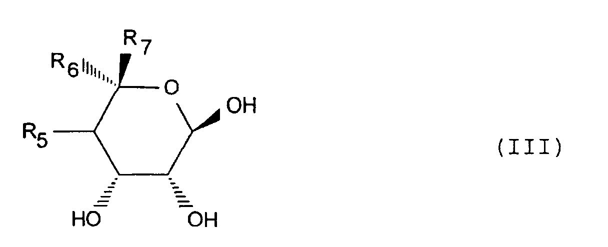 in which R₅, R₆ and R₇ retain their previous meaning on a compound of formula (IV) 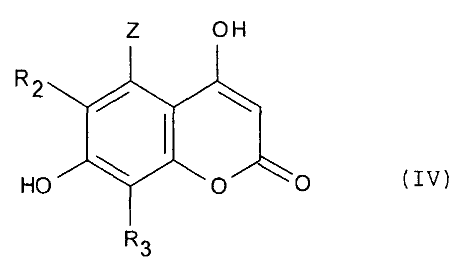 in which R₂, R₃ and Z retain their previous meaning, then of a blocking agent of the free hydroxyl radical.

26. As new chemical products, the following compounds of formula (III) defined in claim 25:

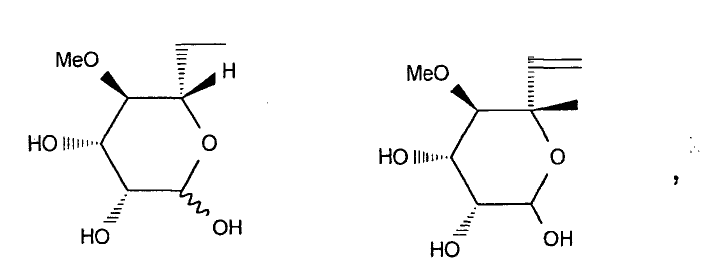

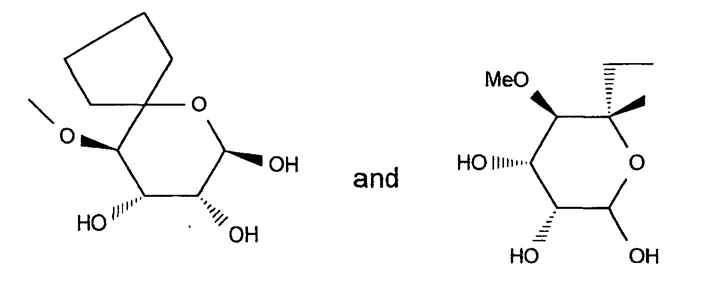

## Patentansprüche

1. Die Verbindungen der Formel (I): 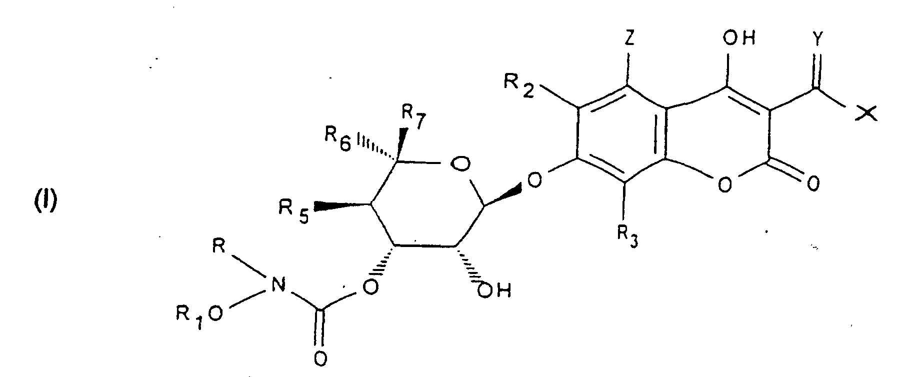 worin:
- Y darstellt ein Sauerstoffatom oder einen Rest N-Nalc₁ oder NOalc₂, worin alc₁ und alc₂ einen Alkylrest mit bis zu 12 Kohlenstoffatomen darstellen, gegebenenfalls unterbrochen durch ein oder mehrere Sauerstoff-, Schwefel- oder Stickstoffatome, gegebenenfalls substituiert mit einem oder mehreren Halogenatomen, mit einem Arylrest, gegebenenfalls substituiert mit einem oder mehreren Halogenatomen, mit einem heterocyclischen Rest, mit einem oder mehreren Resten 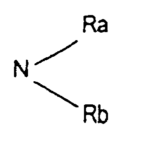
worin Ra und Rb, die gleich oder voneinander verschieden sind, ein Wasserstoffatom, einen Alkylrest mit bis zu 8 Kohlenstoffatomen, gegebenenfalls substituiert, darstellen, oder Ra und Rb zusammen mit dem Stickstoffatom, an das sie gebunden sind, einen Heterocyclus bilden, der außerdem ein weiteres Heteroatom enthalten kann, das ausgewählt ist unter Sauerstoff, Schwefel oder Stickstoff,
- X darstellt ein Wasserstoffatom, einen Hydroxylrest, einen linearen, verzweigten oder cyclischen Alkyl-, Alkenyl- oder Alkinylrest, gegebenenfalls unterbrochen durch ein oder mehrere Sauerstoff-, Schwefel- und/oder Stickstoffatome, mit bis zu 12 Kohlenstoffatomen, gegebenenfalls substituiert mit einem oder mehreren Halogenatomen, mit einem heterocyclischen Rest, einem oder mehreren freien oder
veresterten OH-Resten, C≡N, NO₂, 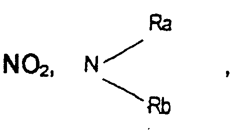 worin Ra und Rb, die gleich oder
verschieden sind, ein Wasserstoffatom, einen Alkylrest mit bis zu 8 Kohlenstoffatomen darstellen, oder Ra und Rb mit dem Stickstoffatom, an das sie gebunden sind, einen Heterocyclus bilden, der gegebenenfalls ein weiteres Heteroatom enthält, das ausgewählt ist unter Stickstoff, Schwefel oder Sauerstoff, oder X darstellt einen Alkoxyrest oder einen Rest 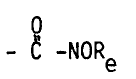
worin Re einen Alkylrest mit bis zu 8 Kohlenstoffatomen darstellt, gegebenenfalls substituiert mit einem oder mehreren der oben angegebenen Substituenten, oder X darstellt einen Rest NRcRd, worin Rc und Rd, die gleich oder verschieden sind, darstellen ein Wasserstoffatom oder einen Alkylrest mit bis zu 12 Kohlenstoffatomen, gegebenenfalls substituiert mit einem oder mehreren der oben angegebenen Substituenten, oder Rc und Rd zusammen mit dem Stickstoffatom, an das sie gebunden sind, einen Heterocyclus bilden, der gegebenenfalls ein weiteres Heteroatom enthält, das ausgewählt ist unter Stickstoff, Schwefel oder Sauerstoff,
- Z ein Wasserstoff- oder Halogenatom oder einen freien, veretherten oder veresterten OH-Rest darstellt,
- R₂ ein Wasserstoff- oder Halogenatom darstellt,
- R₃ ein Wasserstoffatom, einen Alkylrest mit bis zu 8 Kohlenstoffatomen oder ein Halogenatom darstellt,
- R ein Wasserstoffatom oder einen Alkylrest mit bis zu 4 Kohlenstoffatomen darstellt,
- R₁ ein Wasserstoffatom, einen linearen, verzweigten oder cyclischen Alkyl-, Alkenyl- oder Alkinylrest mit bis zu 8 Kohlenstoffatomen darstellt, gegebenenfalls substituiert mit einem oder mehreren Halogenatomen, einem C≡N-Rest, einem Arylrest mit bis zu 14 Kohlenstoffatomen,
- R₅ ein Wasserstoffatom, einen O-Alkylrest mit bis zu 4 Kohlenstoffatomen darstellt,
- entweder R₆ einen Alkyl- oder CH₂O-Alkylrest darstellt, worin Alkyl einen Alkylrest mit bis zu 8 Kohlenstoffatomen, Ethenyl oder 2-Propenyl darstellt,
- R₇ ein Wasserstoffatom oder einen Alkylrest mit bis zu 8 Kohlenstoffatomen darstellt,
- oder aber R₆ und R₇ mit dem Kohlenstoffatom, das sie trägt, einen Ring mit bis zu 8 Kohlenstoffatomen bilden, sowie die Salze der Verbindung der Formel (I), wenn die Verbindungen der Formel (I) eine basische Funktion haben.

2. Die Verbindungen der Formel (I), die in Anspruch 1 definiert sind, worin Y ein Sauerstoffatom darstellt.

3. Die Verbindungen der Formel (I), worin Y einen NO-Alkylrest darstellt, worin der Alkylrest bis zu 4 Kohlenstoffatome enthält.

4. Die Verbindungen der Formel (I), die in Anspruch 3 definiert sind, worin Y den NOC₂H₅-Rest darstellt.

5. Die Verbindungen der Formel (I), die in einem der Ansprüche 1 bis 4 definiert sind, worin X einen Alkylrest mit bis zu 4 Kohlenstoffatomen und insbesondere den CH₃-Rest darstellt.

6. Die Verbindungen der Formel (I), die in einem der Ansprüche 1 bis 4 definiert sind, worin X einen NH₂-Rest darstellt.

7. Die Verbindungen der Formel (I), die in einem der Ansprüche 1 bis 4 definiert sind, worin X den Rest: 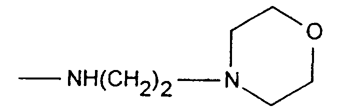 darstellt.

8. Die Verbindungen der Formel (I), die in einem der Ansprüche 1 bis 7 definiert sind, worin R₁ einen Rest:
HC≡C-CH₂-
darstellt.

9. Die Verbindungen der Formel (I), die in einem der Ansprüche 1 bis 8 definiert sind, worin R ein Wasserstoffatom darstellt.

10. Die Verbindungen der Formel (I), die in einem der Ansprüche 1 bis 9 definiert sind, worin R₃ einen Methylrest darstellt.

11. Die Verbindungen der Formel (I), die in einem der Ansprüche 1 bis 10 definiert sind, worin Z ein Wasserstoffatom darstellt.

12. Die Verbindungen der Formel (I), die in einem der Ansprüche 1 bis 11 definiert sind, worin R₂ ein Wasserstoffatom darstellt.

13. Die Verbindungen der Formel (I), die in einem der Ansprüche 1 bis 12 definiert sind, worin R₅ einen OCH₃-Rest darstellt.

14. Die Verbindungen der Formel (I), die in einem der Ansprüche 1 bis 13 definiert sind, worin R₆ einen Methylrest darstellt.

15. Die Verbindungen der Formel (I), die in einem der Ansprüche 1 bis 14 definiert sind, worin R₇ einen Methylrest darstellt.

16. Die Verbindungen der Formel (I), die in einem der Ansprüche 1 bis 14 definiert sind, worin R₇ einen Ethylrest darstellt.

17. Die Verbindungen der Formel (I), die in einem der Ansprüche 1 bis 13 definiert sind, worin R₆ und R₇ mit dem Kohlenstoffatom, das sie trägt, einen Cyclopentylrest bilden.

18. Die Verbindungen der Formel (I), die in Anspruch 1 definiert sind, deren Namen folgen:
- (2-Propinyloxy)carbaminsäure-3'-ester von 7-[[6-Deoxy-5-C-methyl-4-O-methylalpha-L-lyxo-hexopyranosyl]oxy]-4-hydroxy-8-methyl-2-oxo-2H-1-benzopyran-3-carbonsäureamid
- (2-Propinyloxy)carbaminsäure-3'-ester von 7-[(6-Deoxy-5-C-methyl-4-O-methylalpha-L-lyxo-hexopyranosyl)oxy]-4-hydroxy-8-methyl-N-[2-(4-morpholinyl)ethyl]-2-oxo-2H-1-benzopyran-3-carbonsäureamid
- (2-Propinyloxy)carbaminsäure-3'-ester von 7-[[6-Deoxy-5-C-methyl-4-O-methylalpha-L-lyxo-hexopyranosyl]oxy]-4-hydroxy-3-[1-(methoxyimino)ethyl]-8-methyl-2H-1-benzopyran-2-on
- (2-Propinyloxy)carbaminsäure-3'-ester von 7-[(6-Deoxy-5-C-methyl-4-O-methylalpha-L-lyxo-hexopyranosyl)oxy]-3-[1-(ethoxyimino)ethyl]-4-hydroxy-8-methyl-2H-1-benzopyran-2-on.

19. Die Verbindungen der Formel (I), deren Namen folgen:
- (2-Propinyloxy)carbaminsäure-3'-ester von 7-[(6-Deoxy-5-C-ethyl-4-O-methylbeta-D-gulopyranosyl)oxy]-4-hydroxy-3-[1-(methoxyimino)ethyl]-8-methyl-2H-1-benzopyran-2-on
- [7R-(7-alpha-8-beta-9-beta-10-alpha)]-8-Hydroxy-7-[4-hydroxy-3-[1-(methoxyimino)ethyl]-8-methyl-2-oxo-2H-1 -benzopyran-7-yl]-10-methoxy-6-oxaspiro[4.5]-decan-9-yl-(2-propinyloxy)-carbamat.

20. Als Medikamente die Verbindungen der Formel (I), die in einem der Ansprüche 1 bis 18 definiert sind, sowie ihre pharmazeutisch annehmbaren Salze.

21. Als Medikamente die Verbindungen der Formel (I), die in Anspruch 19 definiert sind, sowie ihre pharmazeutisch annehmbaren Salze.

22. Die pharmazeutischen Zusammensetzungen, die als Wirkstoff wenigstens ein Medikament, das in Anspruch 20 oder 21 definiert ist, enthalten.

23. Verfahren zur Herstellung der Verbindungen der Formel (I), die in einem der Ansprüche 1 bis 19 definiert sind, **dadurch gekennzeichnet, dass** man eine Verbindung der Formel (II) 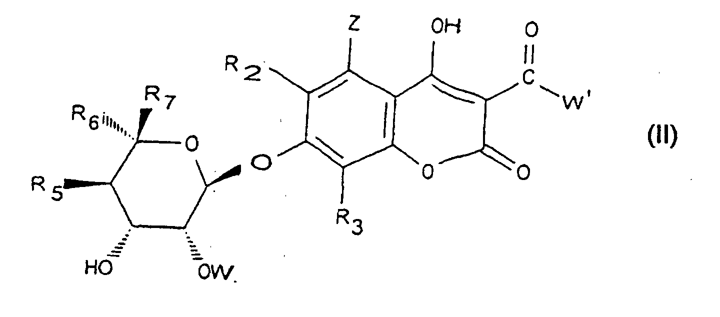 worin die Reste R₂, R₃, Z, R₅, R₆ und R₇ ihre vorherige Bedeutung behalten, OW eine blockierte Hydroxylgruppe darstellt und W einen Alkyl- oder OAlkylrest mit bis zu 4 Kohlenstoffatomen darstellt, unterzieht
- der Einwirkung eines Mittels, das den Rest 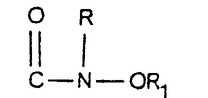 einführen kann
oder einer Abfolge von Arbeitsschritten, die den Rest 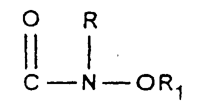 einführen können,
wobei R und R₁ ihre vorherige Bedeutung behalten,
- der Einwirkung eines Mittels, das den Hydroxylrest aus dem Rest OW freisetzen kann,
- der etwaigen Einwirkung eines Mittels, das W durch den Rest X, der von Alkyl oder OAlkyl verschieden ist, ersetzen kann,
- der etwaigen Einwirkung eines Mittels, das den Rest Y, der von Sauerstoff verschieden ist, einführen kann,
- der etwaigen Einwirkung eines Mittels zur Salzbildung.

24. Als neue chemische Produkte die Verbindungen der Formel (II), die in Anspruch 23 definiert sind.

25. Verfahren gemäß Anspruch 23, **dadurch gekennzeichnet, dass** das Produkt der Formel (II) durch Einwirkung einer Verbindung der Formel (III) 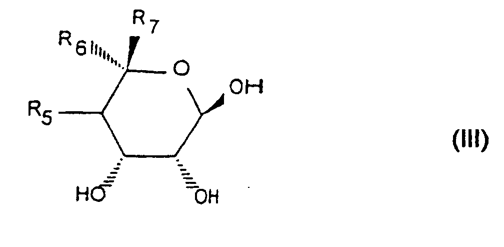 worin R₅, R₆ und R₇ ihre vorherige Bedeutung behalten, auf eine Verbindung der Formel (IV) 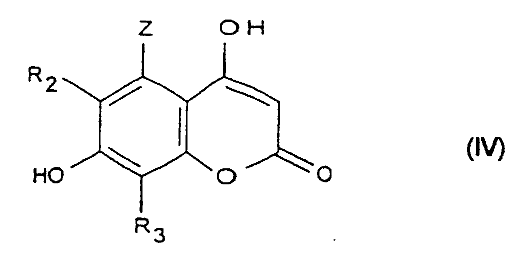 worin R₂, R₃ und Z ihre vorherige Bedeutung behalten, dann eines Mittels zum Blockieren des freien Hydroxylrests hergestellt wird.

26. Als neue chemische Produkte die folgenden Verbindungen der Formel (III), die in Anspruch 25 definiert sind:
